# FASCICULE DE BREVET EUROPEEN

(11) **EP 3 795 134 B1**
(45) Date de publication et mention de la délivrance du brevet: **10.05.2023**
(21) Numéro de dépôt: 19306126.4
(22) Date de dépôt: 19.09.2019
(51) Int. Cl.: A61K 8/06, A61K 8/60, A61K 8/81, A61Q 13/00, C11B 9/00

(54) **COMPOSITIONS PARFUMANTES, STABLES ET POSSÉDANT DE BONNES PROPRIÉTÉS DE PULVÉRISATION**
STABILE DUFTZUSAMMENSETZUNGEN, DIE GUTE SPRÜHEIGENSCHAFTEN BESITZEN
PERFUMING COMPOSITIONS, STABLE AND HAVING GOOD SPRAYING PROPERTIES

(43) Date de publication de la demande: 24.03.2021
(73) Titulaire: Société d'Exploitation de Produits pour les Industries Chimiques SEPPIC, 75321 Paris cedex 07 (FR); Seppic Italia Srl, 20151 Milano (IT)
(72) Inventeur: COLOMBO, Fabrizio, 20151 MILANO (IT); CACCIALANZA, Francesca, 20151 MILANO (IT); GRIMALDI, Giovanni, 20140 MILANO (IT)
(74) Mandataire: Air Liquide

(56) Documents cités:
- WO-A1-02/32390
- WO-A1-2018/109354
- WO-A2-02/100372
- WO-A2-03/035657
- SEPPIC-Air Liquide: "Emulsifier range", , 2018, pages 1-2, XP002798031, Extrait de l'Internet: URL:https://latinamerica.in-cosmetics.com/ __novadocuments/548880?v=63683840255343000 0 [extrait le 2020-03-03]

## Description

L'invention a pour objet de nouvelles compositions parfumantes, stables et possédant de bonnes propriétés de pulvérisation, ainsi qu'un procédé pour parfumer la peau, les cheveux, le cuir chevelu, les lèvres, les vêtements ou les linges de maison mettant en oeuvre les nouvelles compositions parfumantes, et les récipients comprenant lesdites nouvelles compositions parfumantes.

Il existe un besoin récurrent dans les industries cosmétiques et de la parfumerie de développer des compositions parfumantes comprenant une concentration réduite en alcools volatils, et même des compositions parfumantes exemptes d'alcools volatils.

De telles compositions parfumantes montrent de nombreux inconvénients liés à la présence de ces alcools volatils, et plus particulièrement à la présence d'éthanol. En effet, comme ces alcools volatils peuvent provoquer un dessèchement de la peau et irriter l'épiderme, elles sont sensibles à la lumière du soleil et leur utilisation dans le domaine des parfums est difficile car l'odeur de l'alcool volatil peut interférer avec les substances parfumantes.

L'eau est un substitut de choix des alcools volatils dans les compositions parfumantes, car son utilisation permet de diminuer la teneur en composés volatils organiques dans l'atmosphère, de réduire ou d'éliminer le caractère inflammable des compositions parfumantes. Par ailleurs, les industries de la parfumerie et de la cosmétique commercialisent également des compositions nécessitant la présence d'eau, comme les eaux de toilettes, les eaux de parfum, des lotions aqueuses après-rasage, des eaux de soin et des eaux fraîches. Ces produits cosmétiques parfumés aqueux sont appréciés des consommateurs car ils se caractérisent par une sensation de fraîcheur accrue.

Cependant, les substances parfumantes étant de façon générale des substances hydrophobes, il s'avère nécessaire d'utiliser au moins un tiers ingrédient permettant de les solubiliser dans la phase aqueuse des compositions parfumantes. Les tensioactifs sont généralement des agents solubilisants qui sont associés aux substances parfumantes hydrophobes pour préparer des compositions parfumantes aqueuses.

Parmi les tensioactifs couramment utilisés, il y a les tensioactifs non ioniques alcoxylés comme les polysorbates, comme les esters de lauroyl sorbitan éthoxylés à 20 moles d'oxyde d'éthylène , les esters de palmitoyl sorbitan éthoxylés à 20 moles d'oxyde d'éthylène , les esters de stéaroyl sorbitan éthoxylés à 20 moles d'oxyde d'éthylène, les esters d'oléyl sorbitan éthoxylés à 20 moles d'oxyde d'éthylène ; les alcools gras éthoxylés, les acides gras éthoxylés, les esters gras éthoxylés, et plus particulièrement les triglycérides gras éthoxylés comme l'huile de ricin hydrogénée éthoxylée à 20, 40 ou 60 moles d'oxyde d'éthylène, et plus particulièrement l'huile de ricin hydrogénée éthoxylée à 40 moles d'oxyde d'éthylène couramment commercialisée sous l'appellation « PEG-40 hydrogenated castor oil ».

Ces tensioactifs éthoxylés présentent cependant l'inconvénient d'être préparés par la mise en oeuvre d'oxyde d'éthylène, dont les teneurs résiduelles, et les sous-produits qu'ils génèrent, sont proscrits dans les produits cosmétiques à destination des consommateurs, impliquant alors la mise en oeuvre de procédé de purification restrictifs. De plus, les composés polyéthoxylés sont suspectés de passer facilement la barrière cutanée et de permettre ainsi un accès plus rapide de l'oxyde d'éthylène résiduel et de ses sous-produits dans l'organisme humain.

Partant de là un problème qui se pose est de fournir une composition parfumante exempte d'alcools, stable et pulvérisable à la fois.

Une solution de la présente invention est une composition parfumante (C₁) se présentant sous la forme d'une émulsion de type huile-dans-eau et comprenant:
a) Au moins une huile,
b) Au moins un polyélectrolyte anionique réticulé (PA),
c) Au moins un tensioactif émulsionnant de type eau-dans huile (S1) comprenant un alkylpolyxyloside possédant un radical alkyl branché, non linéaire et comportant de 10 à 36 atomes de carbone,
d) Au moins un tensioactif émulsionnant de type huile-dans-eau (S2),
e) Au moins une substance parfumante hydrophobe, et
f) de l'eau;
avec la ratio massique du tensioactif émulsionnant de type huile-dans-eau (S2) sur le tensioactif émulsionnant de type eau-dans-huile (S1) compris entre 2,0/1.

Selon le cas la composition selon l'invention peut présenter une ou plusieurs des caractéristiques suivantes :
- ladite composition comprend au moins une cire
- ladite composition comprend pour 100% de sa masse :
   a) De 1% à 30% massique d'huile, de préférence de 1% à 25% massique d'huile ;
   b) De 0,05% à 2,5% massique du polyélectrolyte anionique réticulé (PA), de préférence de 0,1% à 2,5% massique et encore plus préférentiellement de 0,5% à 2,5% massique du polyélectrolyte anionique réticulé (PA) ;
   c) De 0,05% à 2,5% massique du tensioactif de type eau-dans-huile (S1), de préférence de 0,1% à 2,5% massique et encore plus préférentiellement de 0,5% à 2,5% massique du tensioactif de type eau-dans-huile (S1);
   d) De 0,5% à 5% massique du tensioactif de type huile-dans-eau (S2), de préférence de 1% à 5% massique du tensioactif de type huile-dans-eau (S2);
   e) De 0,5% à 10% massique de la substance parfumante hydrophobe, de 1% à 10% massique d'au moins une substance parfumante hydrophobe;
   f) De 50% à 97,9% massique d'eau, de préférence de 55% à 97,8% massique et encore plus préférentiellement de 55% à 96% massique d'eau.
      De préférence la somme des proportions massiques des constituants (a), (b), (c), (d), (e) et (f) est égale à 100% de la masse de la composition (C1).
- le tensioactif émulsionnant de type eau-dans-huile (S1) comprend au moins une composition d'alkylpolyglycosides (C'1) représentées par la formule (I) :

   R₂-O-(X)ₓ-H (I)

   dans laquelle x représente un nombre décimal compris entre 1,05 et 2,5, X représente le radical xylosyl ou α,β-D-xylopyranosyl, obtenu à partir de la suppression du groupe hydroxyl hémiacétal du α,β-D-xylopyranose, et R2 représente un radical de formule (II) :

      CH (CₜH₂ₜ₊₁)(CᵥH₂ᵥ₊₁)CH₂₋ (II)
   dans laquelle t est un nombre entier compris entre 6 et 18, v est un nombre entier compris entre 4 et 18 et la somme t + v est supérieure ou égale à 10 et inférieure ou égale à 22.
- la composition (C'1) consiste en un mélange de composés représentés par les formules (I1), (I2), (I3), (I4) et (I5) :

   R2-O-(X)1-H (I1)

   R2-O-(X)2-H (I2)

   R2-O-(X)3-H (I3)

   R2-O-(X)4-H (I4)

   R2-O-(X)5-H (I5)

   dans les proportions molaires respectives a1, a2, a3, a4 et a5, telles que :
   ▪ La somme a1+ a2 + a3 + a4 + a5 est égale à 1 et que
   ▪ La somme a1 + 2a2 + 3a3 + 4a4 + 5a5 est égale à x.
- le tensioactif émulsionnant de type eau-dans-huile comprend un alcool gras de formule (III) :

   CH(CₜH₂ₜ₊₁)(CᵥH₂ᵥ₊₁)CH₂-OH (III),

   dans laquelle t est un nombre entier compris entre 6 et 18, v est un nombre entier compris entre 4 et 18 et la somme t + v est supérieure ou égale à 14.
- le tensioactif émulsionnant de type huile-dans-eau (S2)comprend :
   ▪ Un composé de formule (IV)

      R₁-(C=O)-[O-CH₂-CH(OH)-CH₂]ₚ-OH (IV),

      dans laquelle p représente un nombre entier supérieur ou égal à 1 et inférieur ou égal à 15, et dans laquelle le groupe R₁-(C=O)- représente un radical acyle, saturé ou insaturé, linéaire ou branché, comportant de six à vingt-deux atomes de carbone, et/ou
   ▪ Une composition (C'₂) d'alkylpolyglycosides représentée par la formule (VI) :

      R₃-O-(G)x'-H (VI)

      dans laquelle x' représente un nombre décimal compris entre 1,05 et 2,5, G représente le radical glucosyl ou α,β-D-glucopyranosyl, obtenu à partir de la suppression du groupe hydroxyl hémiacétal du α,β-D-glucopyranose, et R₃ représente un radical choisi parmi les éléments du groupe constitué par le radical n-dodécyle, n-tétradécyle, n-hexadécyle, n-octadécyle, n-eicosyle et n-béhényle.
- la composition (C'₂) consiste en un mélange de composés représentés par les formules (VI₁), (VI₂), (VI₃), (VI₄) et (VI₅) :

   R₃-O-(G)1-H (VI1)

   R₃-O-(G)2-H (VI2)

   R₃-O-(G)3-H (VI3)

   R₃-O-(G)4-H (VI4)

   R₃-O-(G)5-H (VI5)

   dans les proportions molaires respectives a1, a2, a3, a4 et a5, telles que :
   ▪ La somme a1+ a2 + a3 + a4 + a5 est égale à 1 et que
   ▪ La somme a1 + 2a2 + 3a3 + 4a4 + 5a5 est égale à x.
- le tensioactif émulsionnant de type huile-dans-eau (S2) comprend un composé de formule (IV) et au moins un composé de formule (V) :

   HO-[CH₂-CH(OH)-CH₂-O]ₙ-H (V),

   Dans laquelle n, identique ou différent de p, représente un nombre entier supérieur ou égal à 1 et inférieur ou égal à 15.
- le tensioactif émulsionnant de type huile-dans-eau (S2) comprend une composition (C'₂) d'alkylpolyglycosides et un alcool gras de formule (VII) :

   R'₃-OH (VII),

   dans laquelle R'₃ représente un radical choisi parmi les éléments du groupe constitué par le radical n-dodécyle, n-tétradécyle, n-hexadécyle, n-octadécyle, n-eicosyle et n-béhényle, et avec R'₃ identique ou différent de R₃.
- le radical R2 représente le radical 2-octyl dodécanyl-1 dans la formule (II) obtenu à partir de la suppression du groupe hydroxyl du 2-octyl dodécanol-1, et pour lequel t est égal à 10 et v est égal à 8 dans la formule (II).
- le polyélectrolyte anionique réticulé (PA) comprend une proportion supérieure ou égale à 25% molaire d'unités monomériques issues de l'acide 2-methy 2-[(1-oxo 2-propènyl) amino] 1-propanesulfonique sous forme acide libre ou partiellement ou totalement salifiée.
- la substance parfumante hydrophobe (A) est choisie parmi les éléments du groupe constitué par les familles olfactives suivantes : Hespéridé, Florale, Fougère, Chypre, Boisée.

Par huile, on désigne, dans la définition de la composition (C₁) objet de la présente invention, un composé et/ou un mélange de composés insoluble dans l'eau, et liquide à 25°C, et plus particulièrement :
- Les alcanes linéaires comportant de onze à dix-neuf atomes de carbone ;
- Les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane), ou des mélanges de certains d'entre eux comme ceux cités ci-après et identifiés par leur nom INCI : C₇₋₈ isoparaffin, C₈₋₉ isoparaffin, C₉₋₁₁ isoparaffin, C₉₋₁₂ isoparaffin, C₉₋₁₃ isoparaffin, C₉₋₁₄ isoparaffin, C₉₋₁₆ isoparaffin, C₁₀₋₁₁ isoparaffin, C₁₀₋₁₂ isoparaffin, C₁₀₋₁₃ isoparaffin, C₁₁₋₁₂ isoparaffin, C₁₁₋₁₃ isoparaffin, C₁₁₋₁₄ isoparaffin, C₁₂₋₁₄ isoparaffin, C₁₂₋₂₀ isoparaffin, C₁₃₋₁₄ isoparaffin, C₁₃₋₁₆ isoparaffin ;
- Les cyclo-alcanes optionnellement substitués par un ou plusieurs radicaux alkyles linéaires ou ramifies ;
- Les huiles blanches minérales, comme celles commercialisées sous les noms suivants : Marcol^{™}52, Marcol^{™}82, Drakeol^{™}6VR, Eolane^{™}130, Eolane^{™}150 ;
- L'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange (M₁) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms Emogreen^{™}L15 ou Emogreen^{™}L19 ;
- Les éthers d'alcool gras de formule (VIII) :

   Z₁-O-Z₂ (VIII),

   dans laquelle Z₁ et Z₂ identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther.
- Les mono-esters d'acides gras et d'alcools de formule (IX) :

   R'₄-(C=O)-O-R'₅ (IX),

   dans laquelle R'₄-(C=O) représente un radical acyle, saturé ou insaturé, linéaire ou ramifié, comportant de huit à vingt-quatre atomes de carbone, et R'₅ représente, indépendamment de R'₄, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l' oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, l'isostéarate d'isostéaryle ;
- Les di-esters d'acides gras et de glycérol de formule (X) et de formule (XI) :

   R'₆-(C=O)-O-CH₂-CH(OH)-CH₂-O-(C=O)-R'₇ (X)

   R'₈-(C=O)-O-CH₂-CH[O-(C=O)-R'₉]-CH₂-OH (XI),

   formules (X) (XI) dans lesquelles R'₆-(C=O), R'₇-(C=O), R'₈-(C=O), R'₉-(C=O), identiques ou différents, représentent un groupement acyle, saturé ou insaturé, linéaire ou ramifié, comportant de huit à vingt-quatre atomes de carbone ;
- Les tri-esters d'acides gras et de glycérol de formule (XII) :

   R'₁₀-(C=O)-O-CH₂-CH[O-(C=O)-R'_{H}]-CH₂-O-(C=O)-R'₁₂ (XII),

   dans laquelle R'₁₀-(C=O), R'₁₁-(C=O) et R'₁₂-(C=O), identiques ou différents, représentent un groupement acyle, saturé ou insaturé, linéaire ou ramifié, comportant de huit à vingt-quatre atomes de carbone.
- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- Les huiles végétales éthoxylées.

Une cire est optionnellement ajoutée à l'huile. Par « cire » présente dans la phase grasse de la composition (C₁) se présentant sous la forme d'une émulsion de type huile-dans-eau telle que définie précédemment, on désigne au sens de la présente invention les substances chimiques ou les mélanges de substances chimiques insolubles dans l'eau, et se présentant sous un aspect solide à une température de 45°C.

La cire est plus particulièrement choisie parmi la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline; l'ozokérite; la cire de polyéthylène; les cires de silicone ; les cires végétales ; les alcools gras et les acides gras solides à température ambiante; les glycérides solides à température ambiante.

Par « tensioactif émulsionnant de type eau-dans-huile», on désigne au sens de la présente invention une substance chimique amphiphile ou une composition chimique de substances chimiques amphiphiles, qui permet de stabiliser une dispersion d'eau ou de phase aqueuse dans une phase grasse continue, comme par exemple une huile et/ou une cire. De tels tensioactifs émulsionnants de type eau-dans-huile sont généralement caractérisés par une valeur de la balance hydrophile/lipophile inférieure ou égale à 8,0, et plus particulièrement inférieure ou égale à 7,0.

Un tel tensioactif émulsionnant de type eau-dans-huile (S₁) est plus particulièrement choisi parmi les éléments du groupe constitué par i) les compositions d'alkylpolyglycosides (C'₁) représentées par la formule (I) telle que définie précédemment et associées ou non à un alcool gras de formule (III) telles que précédemment définies.

Selon un aspect particulier de la présente invention, R₂ représente un radical de formule (II) dans laquelle t est un nombre entier compris entre 6 et 10, v est un nombre entier compris entre 8 et 12 et la somme t + v est supérieure ou égale à 14 et inférieure ou égale à 18.

Selon un aspect particulier de la présente invention, R₂ représente un radical de formule (II) choisi parmi le groupe constitué par le radical 2-hexyl octyle, 2-hexyl décyle, 2-octyl décyle, 2-hexyl dodécyle, 2-octyl dodécyle.

Selon un aspect plus particulier, l'invention a pour objet une composition (C₁) pour laquelle le radical R₂ représente le radical 2-octyl dodécanyl-1 dans la formule (II), obtenu à partir de la suppression du groupe hydroxyl du 2-octyl dodécanol-1, et pour lequel t est égal à 10 et v est égal à 8 dans les formules (II) et (III).

Dans la composition (C'₁), présente dans la composition (C₁) objet de la présente invention, X représente le radical xylosyl ou α,β-D-xylopyranosyl, obtenu à partir de la suppression du groupe hydroxyl hémiacétal du α,β-D-xylopyranose, et la structure oligomérique (X)x peut se présenter sous toutes formes d'isoméries, qu'il s'agisse d'isomérie optique, d'isomérie géométrique ou d'isomérie de position ; elle peut aussi représenter un mélange d'isomères. Dans la formule (I) telle que définie ci-dessus, le groupe Rz-O- est lié à X par le carbone anomérique du reste saccharide, de manière à former une fonction acétal.

Selon un aspect encore plus particulier, dans la définition de la formule (I) représentant la composition (C'₁) comprise dans la composition (C₁) objet de la présente invention, x représente un nombre décimal supérieur ou égal à 1,05 et inférieur ou égal à 2,0, et encore plus particulièrement supérieur ou égal à 1,15 et inférieur ou égal à 2,0.

Selon un aspect encore plus particulier, dans la définition de la formule (I) telle que définie ci-dessus, R₂ représente le radical 2-octyl dodécyle ; X représente le radical xylosyl ou α,β-D-xylopyranosyl, obtenu à partir de la suppression du groupe hydroxyl hémiacétal du α,β-D-xylopyranose et x représente un nombre décimal supérieur ou égal à 1,05 et inférieur ou égal à 2,5.

Selon un aspect particulier le tensioactif émulsionnant de type eau-dans-huile (S1) comprend pour 100% de sa masse :
- De 10% à 50% massique, plus particulièrement de 15% à 40% massique, et encore plus particulièrement de 20% à 30% massique, d'au moins une composition (C'₁) représentée par la formule (I) telle que définie précédemment,
- De 90% à 50% massique, plus particulièrement de 85% à 60% massique, et encore plus particulièrement de 80% à 70% massique, d'au moins un alcool gras de formule (III') :

   R'₂-OH (III'),

   dans laquelle R'₂, identique ou différent de R₂, représente un radical de formule (II) choisi parmi le groupe constitué par le radical 2-hexyl octyle, 2-hexyl décyle, 2-octyl décyle, 2-hexyl dodécyle, 2-octyl dodécyle.

Selon un aspect particulier, le radical R'₂ de la formule (III') est identique au radical R₂ de la formule (I).

Selon un autre aspect plus particulier, le tensioactif émulsionnant de type eau-dans-huile (S1) comprend pour 100% de sa masse :
- De 10% à 50% massique, plus particulièrement de 15% à 40% massique, et encore plus particulièrement de 20% à 30% massique, d'au moins une composition (C'₁) représentée par la formule (I) pour laquelle , R₂ représente le radical 2-octyl dodécyle ; X représente le radical xylosyl ou α,β-D-xylopyranosyl, obtenu à partir de la suppression du groupe hydroxyl hémiacétal du α,β-D-xylopyranose et x représente un nombre décimal supérieur ou égal à 1,05 et inférieur ou égal à 2,5,
- De 90% à 50% massique, plus particulièrement de 85% à 60% massique, et encore plus particulièrement de 80% à 70% massique, d'au moins un alcool gras de formule (III') pour laquelle R'₂, identique à R₂, représente le radical 2-octyl dodécyle.

Selon un autre aspect particulier, la composition (C₁) objet de la présente invention comprend pour 100% de sa masse, de 0,5% à 5% massique, et encore plus particulièrement de 1% à 5% massique d'au moins un tensioactif émulsionnant de type huile-dans-eau (S₂) tel que défini précédemment.

Par « tensioactif émulsionnant de type huile-dans-eau », on désigne au sens de la présente invention une substance chimique amphiphile ou une composition chimique de substances chimiques amphiphiles, qui permet de stabiliser une dispersion d'une phase grasse, comme par exemple une huile et/ou une cire, dans une phase aqueuse continue.

De tels tensioactifs émulsionnants de type huile-dans-eau (S₂) sont généralement caractérisés par une valeur de la balance hydrophile/lipophile supérieure à 8,0, et plus particulièrement inférieure ou égale à 20,0.

Un tel tensioactif émulsionnant de type huile-dans-eau (S₂) est plus particulièrement choisi parmi les éléments du groupe constitué par i) un composé de formule (IV), ii) une composition (C₂) d'au moins un composé de formule (IV) et d'au moins un composé de formule (V), iii) une composition (C'2) d'alkylpolyglycosides représentée par la formule (VI) associée ou non à un alcool gras de formule (VII).

Selon un aspect particulier, la présente invention a pour objet une composition (C₁) pour laquelle, dans la formule (IV), p représente un nombre entier supérieur ou égal à 2 et inférieur ou égal à 15, plus particulièrement supérieur ou égal à 2 et inférieur ou égal à 10, et encore plus particulièrement supérieur ou égal à 4 et inférieur ou égal à 10.

Selon un autre aspect particulier, la présente invention a pour objet une composition (C₁) pour laquelle, dans la formule (IV), le groupe R₁-(C=O)- représente un radical acyle, saturé ou insaturé, linéaire ou branché, comportant de dix à vingt-deux atomes de carbone, et plus particulièrement un radical choisi parmi les radicaux n-décanoyle, n-dodécanoyle, n-tétradécanoyle, n-hexadécanoyle, n-octadécanoyle, n-eicosanoyle, n-docosanoyle, n-oléyle, n-linoléyle, n-linolénoyle ou isostéaryle.

Selon un autre aspect plus particulier, la présente invention a pour objet une composition (C₁) pour laquelle, dans la formule (IV), p représente un nombre entier supérieur ou égal 4 et inférieur ou égal à 10, et le groupe R₁-(C=O)- représente un radical acyle choisi parmi les membres du groupe comprenant le radical n-décanoyle, le radical n-dodécanoyle, le radical n-tétradécanoyle, le radical n-hexadécanoyle, le radical n-octadécanoyle, le radical n-eicosanoyle, le radical n-docosanoyle.

Selon un autre aspect particulier, la présente invention a pour objet une composition (C₁) pour laquelle, dans la formule (V), n, identique ou différent de p, représente un nombre entier supérieur ou égal à 2 et inférieur ou égal à 15, plus particulièrement supérieur ou égal à 2 et inférieur ou égal à 10, et encore plus particulièrement supérieur ou égal à 4 et inférieur ou égal à 10.

Selon un autre aspect particulier, la présente invention a pour objet une composition (C₁) pour laquelle, dans la formule (V), n identique ou à p, représente un nombre entier supérieur ou égal à 4 et inférieur ou égal à 10.

Dans la définition de la formule (VI) représentant la composition (C'₂) comprise dans la composition (C₁) objet de la présente invention, , G représente le radical glucosyl ou α,β-D-glucopyranosyl, obtenu à partir de la suppression du groupe hydroxyl hémiacétal du α,β-D-glucopyranose,, et la structure oligomérique (G)x' peut se présenter sous toutes formes d'isoméries, qu'il s'agisse d'isomérie optique, d'isomérie géométrique ou d'isomérie de position ; elle peut aussi représenter un mélange d'isomères. Dans la formule (VI) telle que définie ci-dessus, le groupe R₃-O- est lié à G par le carbone anomérique du reste saccharide, de manière à former une fonction acétal.

Selon un aspect plus particulier, dans la définition de la formule (VI) représentant la composition (C'₂) comprise dans la composition (C₁) objet de la présente invention, x' représente un nombre décimal supérieur ou égal à 1,05 et inférieur ou égal à 2,0, et encore plus particulièrement supérieur ou égal à 1,15 et inférieur ou égal à 2,0.

Selon un autre aspect plus particulier, dans la définition de la formule (VI) représentant la composition (C'₂) comprise dans la composition (C₁) objet de la présente invention, R₃ représente un radical choisi parmi les éléments du groupe constitué par le radical n-dodécyle, le radical n-tétradécyle, le radical n-hexadécyle.

Selon un autre aspect encore plus particulier, dans la définition de la formule (VI) représentant la composition (C'₂) comprise dans la composition (C₁) objet de la présente invention, R₃ représente un radical choisi parmi les éléments du groupe constitué par le radical n-dodécyle, le radical n-tétradécyle, le radical n-hexadécyle, et x' représente un nombre décimal supérieur ou égal à 1,05 et inférieur ou égal à 2,5, plus particulièrement supérieur ou égal à 1,05 et inférieur ou égal à 2,0, et encore plus particulièrement supérieur ou égal à 1,15 et inférieur ou égal à 2,0.

Selon un autre aspect, dans la composition (C₁) objet de la présente invention, le tensioactif émulsionnant de type huile-dans-eau (S₂) comprend une composition d'alkylpolyglycosides (C'₂) représentées par la formule (VI) et un alcool gras de formule (VII) telle que définie précédemment.

Selon un autre aspect plus particulier, dans la composition (C₁) objet de la présente invention, le tensioactif émulsionnant de type huile-dans-eau (S₂) comprend pour 100% de sa masse :
- De 10% à 50% massique, plus particulièrement de 15% à 40% massique, et encore plus particulièrement de 20% à 30% massique, d'au moins une composition (C'₂) représentée par la formule (VI) pour laquelle, R₃ représente au moins un radical choisi parmi les éléments du groupe constitué par le radical n-dodécyle, le radical n-tétradécyle, le radical n-hexadécyle, G représente le radical glucosyl ou α,β-D-glucopyranosyl, obtenu à partir de la suppression du groupe hydroxyl hémiacétal du α,β-D-glucopyranose, et x' représente un nombre décimal supérieur ou égal à 1,05 et inférieur ou égal à 2,0,
- De 90% à 50% massique, plus particulièrement de 85% à 60% massique, et encore plus particulièrement de 80% à 70% massique, d'au moins un alcool gras de formule (VII) pour laquelle R'₃, identique à R₃, représente au moins un radical choisi parmi les éléments du groupe constitué par le radical n-dodécyle, le radical n-tétradécyle, le radical n-hexadécyle.

Par polyélectrolyte anionique réticulé (PA), on désigne, dans la définition de la composition (C₁) à usage topique objet de la présente invention, un polyélectrolyte anionique réticulé non linéaire, se présentant à l'état de réseau tridimensionnel insoluble dans l'eau, mais gonflable à l'eau et conduisant à l'obtention d'un gel chimique.

Selon un aspect particulier, dans la composition (C₁) objet de la présente invention, le polyélectrolyte anionique réticulé (PA) comprend pour 100% molaire, une proportion supérieure ou égale à 25% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée.

Selon un aspect particulier, le polyélectrolyte anionique réticulé (PA) comprend pour 100% molaire :
(a1) - une proportion supérieure ou égale à 25% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée ;
(a2) - optionnellement, une proportion supérieure à 0% molaire et inférieure ou égale à 75% molaire, d'unités monomériques issues d'au moins un monomère choisi parmi les éléments du groupe constitué par l'acrylamide, le N,N-diméthyl acrylamide ; le méthacrylamide ou le N-isopropyl acrylamide ;
(a3) - optionnellement une proportion supérieure à 0% molaire et inférieure ou égale à 20% molaire, plus particulièrement supérieure à 0% molaire et inférieure ou égale à 15% molaire, encore plus particulièrement supérieure ou égale 0% molaire et inférieure ou égale à 10% molaire d'unités monomériques issues d'au moins un monomère choisi parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), et le vinyl pyrrolidone ;
(a4) - optionnellement une proportion supérieure à 0% molaire et inférieure ou égale à 75% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3- [(1-oxo 2-propènyl) amino] butanoïque, la fonction carboxylique desdits monomères étant sous forme acide libre, partiellement salifiée ou totalement salifiée ;
(a5) optionnellement une proportion supérieure à 0% molaire et inférieure ou égale à 5% molaire d'au moins un monomère de formule (M1) : 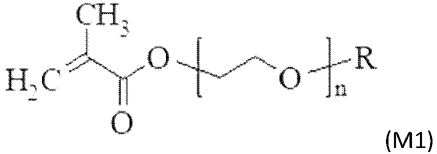 dans laquelle R représente un radical alkyle linéaire ou ramifié comportant de huit à vingt atomes de carbone et n représente un nombre entier supérieur ou égal à zéro et inférieur ou égal à vingt ;
(a6) - d'une proportion supérieure à 0% molaire et inférieure ou égale à 1% molaire d'unités monomériques issues d'au moins un monomère de réticulation (AR) diéthylénique ou polyéthylénique ; la somme des dites proportions molaires en unités monomériques selon a1), a2), a3), a4), a5) et a6) étant égale à 100% molaire.

Au sens de la présente invention, le terme « salifié » indique que la fonction acide présente dans un monomère se trouve sous une forme anionique associée sous forme de sel à un cation, notamment les sels de métaux alcalins, tels que les cations du sodium ou du potassium, ou comme les cations de base azotés tels que le sel d'ammonium, le sel de lysine ou le sel de monoéthanolamine (HOCH₂-CH₂-NH₄⁺). Il s'agit de préférence des sels de sodium ou d'ammonium.

Par au moins un monomère de réticulation (AR) diéthylènique ou polyéthylénique, on désigne, dans la définition dudit polyélectrolyte anionique réticulé (PA), on désigne notamment un monomère choisi parmi les éléments du groupe constitué par le méthylène-bis(acrylamide), le diméthacrylate d'éthylèneglycol, le diacrylate de diéthylèneglycol, le diacrylate d'éthylèneglycol, le diallyl urée, le triallylamine, le triméthylol propanetriacrylate, l'acide diallyoxyacétique ou un de ses sels comme le diallyloxyacétate de sodium, ou un mélange de ces composés ; et plus particulièrement un monomère choisi parmi le diméthacrylate d'éthylèneglycol, le triallylamine, le triméthylol propanetriacrylate ou le méthylène-bis(acrylamide) ou un mélange ces composés.

Selon un autre aspect particulier de la présente invention, la composition (C₁) est caractérisée en ce que ledit monomère de réticulation (AR) tel que défini précédemment, est mis en oeuvre dans une proportion molaire inférieure ou égale à 0,5%, plus particulièrement inférieure ou égale à 0,25% et tout particulièrement inférieure ou égales à 0,1% ; elle est plus particulièrement supérieure ou égales à 0,005% molaire.

Le polyélectrolyte anionique réticulé (PA) mis en oeuvre dans la composition (C₁) à usage topique objet de la présente invention peut également comprendre divers additifs, tels que des agents complexants, des agents de transfert ou des agents limiteurs de chaîne.

Le polyélectrolyte anionique réticulé (PA) mis en oeuvre dans la composition (C₁) à usage topique objet de la présente invention peut être préparé par la mise en oeuvre d'un procédé de polymérisation radicalaire, comme par exemple les procédés de polymérisation en solution, de polymérisation en suspension, de polymérisation en suspension inverse, de polymérisation en émulsion, de polymérisation en émulsion inverse, de polymérisation en milieu solvant suivie d'une étape de précipitation du polymère formé.

Selon un aspect plus particulier, le polyélectrolyte anionique réticulé (PA) mis en oeuvre dans la composition (C₁) à usage topique objet de la présente invention peut être préparé par la mise en oeuvre d'un procédé de polymérisation en milieu solvant suivie d'une étape de précipitation du polymère formé, ou de polymérisation en émulsion inverse optionnellement suivi d'une étape de concentration et/ou d'atomisation.

Selon un aspect plus particulier, le polyélectrolyte anionique réticulé (PA) mis en oeuvre dans la composition (C₁) à usage topique objet de la présente invention peut être préparé selon un des procédés décrits ci-dessus et impliquer l'utilisation d'agents de transfert ou limiteurs de chaîne. Les agents de transfert ou limiteurs de chaîne sont plus particulièrement choisi parmi le groupe constitué par l'hypophosphite de sodium, des alcools de faibles poids moléculaires par exemple le méthanol, l'éthanol, le propanol-1, l'isopropanol, le butanol, des thiols, par exemple le 2-mercapto éthanol, des agents de transfert comprenant une fonction sulfate, par exemple le methallylsulfonate de sodium, ou des mélanges desdits agents de transfert. Les agents de transfert ou limiteurs de chaînes sont plus particulièrement utilisés dans des proportions molaires, exprimées par rapport au nombre total de moles de monomères mis en oeuvre, de 0,001% à 1% molaire, plus particulièrement de 0,001% à 0,5% molaire, et tout particulièrement de 0,001% à 0,1% molaire.

Selon un autre aspect particulier de la présente invention, ledit polyélectrolyte anionique réticulé (PA) est un élément du groupe constitué par un homopolymère de l'acide 2-méthyl 2-[(1-oxo 2-propènyl) amino] 1-propanesulfonique partiellement ou totalement salifié sous forme de sel de sodium ou de sel d'ammonium, réticulé par le triallylamine et/ou le méthylène-bis(acrylamide) ; un copolymère de l'acide 2-méthyl 2-[(1-oxo 2-propènyl) amino] 1-propanesulfonique partiellement ou totalement salifié sous forme de sel de sodium ou de sel d'ammonium et de l'acide acrylique partiellement ou totalement salifiés sous forme de sel de sodium ou de sel d'ammonium, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium ou de sel d'ammonium et d'acide acrylique (δ) partiellement ou totalement salifié sous forme de sel de sodium dans un rapport molaire (γ)/(δ) supérieur ou égal à 30/70 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'acide acrylique (δ)partiellement ou totalement salifié sous forme de sel de sodium dans un rapport molaire (γ)/(δ)supérieur ou égal à 40/60 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'acrylamide (ε) dans un rapport molaire (γ)/(ε) supérieur ou égal à 30/70 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'hydroxyéthylacrylate (ζ) dans un rapport molaire (γ)/(ζ) supérieur ou égal à 30/70 et inférieur ou égal à 90/10, réticulé par le triallylamine et/ou le méthylène-bis(acrylamide) ; un terpolymère réticulé par le triallylamine et/ou le méthylènebis(acrylamide), de l'acide 2-méthyl 2-[(1-oxo 2-propènyl) amino] 1-propanesulfonique partiellement ou totalement salifié sous forme de sel de sodium ou de sel d'ammonium, de l'acrylamide et de l'acide acrylique partiellement ou totalement salifiés sous forme de sel de sodium ou de sel d'ammonium ; un terpolymère réticulé par le triallylamine et/ou le méthylène-bis(acrylamide), de l'acide 2-méthyl 2-[(1-oxo 2-propènyl) amino] 1- propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 45% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 10%, un terpolymère réticulé par le triallylamine et/ou le méthylène-bis(acrylamide), de l'acide 2-méthyl 2-[(1-oxo 2-propènyl) amino] 1-propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 47% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 8% ; un terpolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39,5% et du méthacrylate de lauryle tétraéthoxylé dans une proportion molaire supérieure ou égale à 0,5% et inférieure ou égale à 5% ; un tétrapolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39 %, du méthacrylate de lauroyle dans une proportion molaire supérieure ou égale à 0,5% et inférieure ou égale à 2,5%, et du méthacrylate de stéaroyle dans une proportion molaire supérieure ou égale à 0,5% et inférieure ou égale à 2,5%.

Par substance parfumante hydrophobe (A) on désigne, dans la composition (C1) telle que définie ci-dessus, toute parfum hydrophobe susceptible de dégager une odeur plus ou moins persistante, ledit parfum appartenant à l'une des familles olfactives telles qu'établies par la société française des parfumeurs.

Cette classification consiste en la liste des familles d'odeurs suivantes : famille Hespéridée, famille Florale, famille Fougère, famille Chypre, famille Boisée, famille Ambrée-orientale et famille Cuir.

Selon un autre aspect particulier, la présente invention a pour objet une composition (C₁) pour laquelle, la substance parfumante hydrophobe (A) est choisie parmi les éléments du groupe constitué par les familles olfactives suivantes : Hespéridé, Florale, Fougère, Chypre, Boisée.

L'association de différentes substances parfumantes hydrophobes permet de produire une signature olfactive agréable, plus ou moins persistante.

Selon un aspect plus particulier, la présente invention a pour objet une composition (C₁) pour laquelle, la substance parfumante hydrophobe (A) est choisie parmi au moins un des éléments du groupe constitué par l'Hespéridé Fleuri Chypré, le Fleuri Boisé Fruité, le Fleuri Fruité, la Fougère Fruitée, le Chypre Fruité, le Chypre Fleuri, le Boisé Fruité, l'Ambré Fleuri épicé, l'Ambré Fleuri Fruité.

La substance parfumante hydrophobe (A) peut être choisie parmi des substances parfumantes hydrophobes d'origine naturelle, animale, ou végétale, ou d'origine synthétique.

Parmi les substances parfumantes hydrophobes d'origine animale, il y a le musc, le castoreum, la civette, l'ambre gris, l'absolue de cire d'abeille, l'hyraceum.

Les substances parfumantes hydrophobes d'origine végétale peuvent être extraites de différentes parties des végétaux, comme de la fleur, de la feuille, de la tige, de l'écorce, du bois, de la mousse de bois, du fruit, du bourgeon, de la graine, de la racine, d'herbes, de résines de végétaux ou de lichens.

Parmi les substances parfumantes hydrophobes d'origine végétale issues de l'extraction de fleurs, il y a celles issues de l'extraction de fleurs de roses, de fleurs de jasmin, de fleurs de tubéreuses, de fleurs de champaca, de fleurs de frangipanier (Ylang-ylang), de fleurs de lotus, de fleurs de mimosa, de fleurs d'oeillets, de fleurs d'osmanthus, de fleur d'acacia, de fleurs d'oranger, de fleurs de narcisses, de fleurs de lavandes, de fleurs de gardénias.

Parmi les substances parfumantes hydrophobes d'origine végétale issues de l'extraction de bourgeons, il y a celles issues de l'extraction de bourgeons de cassis.

Parmi les substances parfumantes hydrophobes d'origine végétale issues de l'extraction de mousses, il y a celles issues de l'extraction de mousses de chêne ou de mousses de hêtre.

Parmi les substances parfumantes hydrophobes d'origine végétale issues de l'extraction de feuilles, il y a les substances parfumantes hydrophobes issues de l'extraction de feuilles d'acacia, de feuilles de basilic, de feuilles de valériane, de feuilles de gentiane, de feuilles de violette, de feuilles de géranium, de feuilles de labdanum, de feuilles de romarin, de feuilles de patchouli, de feuilles de verveine.

Parmi les substances parfumantes hydrophobes d'origine végétale issues de l'extraction d'écorce, il y a les substances parfumantes hydrophobes issues de l'extraction d'écorce de cannelle, d'écorce de frêne, d'écorce de cassia, d'écorce de cascarille, d'écorce de bouleau.

Parmi les substances parfumantes hydrophobes d'origine végétale issues de l'extraction du bois, il y a les substances parfumantes hydrophobes issues de l'extraction du bois de santal, du bois de cèdre, du bois de palissandre, du bois d'aloès, du bois de gaïac.

Parmi les substances parfumantes hydrophobes d'origine végétale issues de l'extraction de résines, il y a les substances parfumantes hydrophobes issues de l'extraction de la résine de labdanum, de la résine d'élémi, du baume du Pérou, du baume du tolu, de la résine de Benjoin, de la résine de l'arbre à myrrhe.

Parmi les substances parfumantes hydrophobes d'origine végétale issues de l'extraction de résines, il y a l'encens, l'opoponax, du guggul.

Parmi les substances parfumantes hydrophobes d'origine végétale issues de l'extraction d'herbes ou de graminés, il y a les substances parfumantes hydrophobes issues de l'extraction de l'estragon, de la sauge, du thym, du basilic, du « lemon grass ».

Parmi les substances parfumantes hydrophobes d'origine végétale issues de l'extraction de racines, il y a les substances parfumantes hydrophobes issues de l'extraction de racines d'angélique, de céleri, de cardamone, d'iris, d'acore, de cactus, de vétiver.

Parmi les substances parfumantes hydrophobes d'origine végétale issues de l'extraction de fruits ou de graines, il y a les substances parfumantes hydrophobes issues de l'extraction de gousses de vanille, de graines de coriandre, de graines de badiane, de graines de fenouil, de baies de genièvre, de graines de cardamone, de graines de cumin, de clous de girofle, de fèves de tonka, d'amandes amères, d'agrumes comme de citron jaune, de citron vert, d'orange, de mandarine, de bergamote.

Parmi les substances parfumantes hydrophobes d'origine végétale, il y a les extraits, les absolues, les alcoolats et les huiles essentielles.

Par huile essentielle, on désigne dans la présente demande un produit odorant, répondant à la définition retenue par la norme ISO 9235, généralement de composition complexe, obtenu à partir d'une matière première végétale botaniquement définie, soit par entrainement à la vapeur d'eau, soit par distillation sèche, soit par un procédé mécanique approprié sans chauffage. L'huile essentielle est généralement séparée de la phase aqueuse par un procédé physique n'entraînant pas de changement significatif de sa composition. La matière première végétale mise en oeuvre pour l'obtention de l'huile essentielle peut être fraîche, flétrie, sèche, entière, contuse ou pulvérisée. L'huile essentielle peut en outre subir une étape de procédé ultérieure appropriée, comme une étape de procédé ultérieure permettant d'aboutir à une huile essentielle déterpénée, désesquiterpénée, rectifiée, privée d'un constituant identifié et nommé, décolorée.

Par huile essentielle déterpénée, on désigne une huile essentielle, telle que définie ci-dessus, privée partiellement ou totalement d'hydrocarbures monoterpéniques.

Par huile essentielle désesquiterpénée, on désigne une huile essentielle, telle que définie ci-dessus, privée partiellement ou totalement d'hydrocarbures monoterpéniques et sesquiterpéniques.

Par huile essentielle rectifiée, on désigne une huile essentielle, telle que définie ci-dessus, qui a subi une distillation fractionnée dans le but de supprimer certains constituants ou d'en modifier la teneur.

Les huiles essentielles telles que définies ci-dessus comprennent un mélange de molécules différentes, et lesdites huiles essentielles peuvent être classées dans différentes familles en fonction de la nature chimique de leurs composants majoritaires. Ainsi, on peut définir :
- La classe des huiles essentielles riches en carbures terpéniques et en carbures sesquiterpéniques,
- La classe des huiles essentielles riches en composés portant une fonction alcool ou monoterpinol,
- La classe des huiles essentielles riches en composés portant une fonction ester,
- La classe des huiles essentielles riches en composés portant une fonction aldéhyde,
- La classe des huiles essentielles riches en composés portant une fonction cétone,
- La classe des huiles essentielles riches en composés portant une fonction phénolique,
- La classe des huiles essentielles riches en composés portant une fonction éther,
- La classe des huiles essentielles riches en oxydes sesquiterpéniques,
- La classe des huiles essentielles riches en composés portant au moins un atome de soufre,

Par huiles essentielles riches en carbures terpéniques et en carbures sesquiterpéniques, on désigne les huiles essentielles comprenant pour 100% de leur masse, une proportion massique supérieure ou égale à 35 % desdits carbures terpéniques et carbures sesquiterpéniques.

Les carbures terpéniques et sesquiterpéniques sont des composés chimiques dérivés de l'oligomérisation de l'isoprène.

Parmi les carbures terpéniques couramment présents dans les huiles essentielles, il y a par exemple le limonène, l'α-pinène, le β-pinène, le γ-pinène, le sabinène, le myrcène, le terpinolène, le camphène, le cadinène, le (Z)-β-ocimène, le (E)-β-ocimène, l'α-terpinène, le β-terminène, le γ-terpinène ou le para-cymène.

Parmi les carbures sesquiterpéniques couramment présents dans les huiles essentielles, il y a par exemple le (E)-β-farnésène, le chamazulène, l'α-farnésène,le germacrène-D, le β-caryophyllène, le β-bourbonène, le bicyclogermacrène, le β-élémène, l'α-humulène, le γ-cadinène, le delta-cadinène, l'α-bulnésène, l'α-guaiène, le seychellène, l'α-patchoulène, le β-patchoulène ou le β-élémène.

Parmi les huiles essentielles riches en carbures terpéniques et sesquiterpéniques, il y a celle de térébenthine, de genévrier, de citron, de Patchouli, de Rhododendron, de Verge d'Or, d'Ylang-Ylang III ou d'Ylang-Ylang complète.

Par huiles essentielles riches en composés portant une fonction alcool, on désigne les huiles essentielles comprenant pour 100% de leur masse, une proportion massique supérieure ou égale à 35 % de composés portant une fonction alcool.

Parmi les composés portant une fonction alcool couramment présents dans les huiles essentielles, il y a les monoterpénols, comme le linalol, le géraniol, le menthol, le néomenthol, le cis-hydrate de sabinène, le citronnellol, l'α-terpinéol, les sesquiterpénols comme le viridiflorol, le trans-nérolidol, l'α-cadinol, le τ-cadinol, le trans-farnesol, le cis-farnésols, l'α-bisabolol, le patchoulol ou le pogostol.

Parmi les huiles essentielles riches en composés portant une fonction alcool, il y a celle de Géranium de type Bourbon, de Géranium Egypte, de lavande Aspic, de Rosalina, de coriandre, de bois de rose, de fleurs de rose, de Palmarosa, de menthe poivrée, de monarde fistuleuse, de Lavandin Super, de Lavandin Grosso, ou de Cabreuva.

Par huiles essentielles riches en composés portant une fonction ester, on désigne les huiles essentielles comprenant pour 100% de leur masse, une proportion massique supérieure ou égale à 35 % de mélanges de composés portant une fonction alcool et de composés portant une fonction ester.

Parmi les composés portant une fonction ester couramment présents dans les huiles essentielles, il y a les esters terpéniques comme l'acétate de lynalyle, de menthyle, de néomenthyle, de géranyle, le formate de citronnellyle, l'angélate d'isobutyle, l'angélate d'isoamyle, l'angélate de méthallyle, l'angélate de propyle ou l'angélate de butyle, les esters phénoliques comme l'acétate d'eugényle, les esters aromatiques comme le salicylate de méthyle.

Parmi les huiles essentielles riches en composés portant une fonction ester, il y a celle de Gaulthérie (wintergreen), de Camomille Romaine, de Lavande vraie de Bulgarie, ou de lavande fine.

Par huiles essentielles riches en composés portant une fonction aldéhyde, on désigne les huiles essentielles comprenant pour 100% de leur masse, une proportion massique supérieure ou égale à 35 % de composés portant une fonction aldéhyde.

Parmi les composés portant une fonction aldéhyde couramment présents dans les huiles essentielles, il y a les aldéhydes terpéniques comme le géranial, le néral, l'iso-géranial, l'iso-néral, le citronellal, le citral ou le citronellal, les aldéhydes aromatiques comme l'aldéhyde cinnamique.

Parmi les huiles essentielles riches en composés portant une fonction aldéhydes, il y a celle de cannelle, de citronelle, d'eucalyptus citronné ou d'eucalyptus citriodora.

Par huiles essentielles riches en composés portant une fonction cétone, on désigne les huiles essentielles comprenant pour 100% de leur masse, une proportion massique supérieure ou égale à 35 % de composés portant une fonction cétone.

Parmi les composés portant une fonction cétone couramment présents dans les huiles essentielles, il y a par exemple le camphre, la carvone, la thuyone ou la menthone.

Parmi les huiles essentielles riches en composés portant une fonction cétone, il y a celle de carvi, de sauge, de thuya, d'aneth odorant, de camphrier, ou de menthe verte.

Par huile essentielle riche en composés portant une fonction phénolique, on désigne les huiles essentielles comprenant pour 100% de leur masse, une proportion massique supérieure ou égale à 35 % de composés portant une fonction phénolique.

Parmi les composés portant une fonction phénolique couramment présents dans les huiles essentielles, il y a par exemple le thymol, le carvacol, l'eugénol, l'iso-eugénol ou l'estragol.

Parmi les huiles essentielles riches en composés portant une fonction phénolique, il y a celle de thym, de sarriette, d'origan, de cannelle de Ceylan, de basilic, ou de clou de girofle.

Par huiles essentielles riches en composés portant une fonction éther, on désigne les huiles essentielles comprenant pour 100% de leur masse, une proportion massique supérieure ou égale à 35 % de composés portant une fonction éther.

Parmi les composés portant une fonction éther couramment présents dans les huiles essentielles, il y a par exemple l'anéthol ou l'eucalyptol.

Parmi les huiles essentielles riches en composés portant une fonction éther, il y a celle d'anis vert, de badiane, de fenouil, d'eucalyptus globulus, de fenouil doux ou de niaouli.

Par huiles essentielles riches en oxydes sesquiterpéniques, on désigne les huiles essentielles comprenant pour 100% de leur masse, une proportion massique supérieure ou égale à 35 % de composés de type oxydes sesquiterpéniques.

Parmi les composés de type oxydes sesquiterpéniques couramment présents dans les huiles essentielles, il y a par exemple l'oxyde d'α-bisabolol A, l'oxyde d'α-bisabolol B, l'oxyde d'α-bisabolone, le 1,8-cinéole, l'ascaridol ou l'allicine.

Parmi les huiles essentielles riches en composés de type oxydes sesquiterpéniques, il y a celle de camomille Allemande, d'Hélichryse de Madagascar, de Cajeput ou de chénopode.

Par huiles essentielles riches en composés portant au moins un atome de soufre, on désigne les huiles essentielles comprenant pour 100% de leur masse, une proportion massique supérieure ou égale à 35 % de composés portant au moins un atome de soufre.

Parmi les composés portant au moins un atome de soufre couramment présents dans les huiles essentielles, il y a par exemple le disulfure de diallyle, le trisulfure de diallyle, le tétrasulfure de diallyle, le sulfure de diallyle, le trisulfure de méthyl-allyle ou le disulfure de méthyl-allyle.

Parmi les huiles essentielles riches en composés portant au moins un atome de soufre, il y a par exemple celles de crucifères, de liliacées ou d'ail.

Les substances parfumantes hydrophobes d'origine synthétique sont réaction chimique entre au moins deux composés chimiques, eux-mêmes d'origine naturelle ou synthétique.

Parmi les substances parfumantes hydrophobes d'origine synthétique il y a des hydrocarbures terpéniques (monoterpènes et sesquiterpènes) tels que les myrcènes, les limonènes, les pinènes, le camphène, le cadinène, le cedrène, le farnesène, le caryophyllène, le chamazulène, le 1,1-diméthoxy-2,2,5-triméthyl-4-hexène, les limonènes, le curcumène, le crythmène, les himachelènes, le limonène, le paracymène, les terpinènes et terpinolènes ou les vetivènes,

Parmi les substances parfumantes hydrophobes d'origine synthétique il y a des esters tels que :
- Les acétates de benzyle, de bornyle, de citronellyle, de cédryle, de dihydromyrcényle, de diméthyl-benzylcarbinyle, d'éthyle, de farnésyle, de fenchyle, d'hexyle, de géranyle, d'isobutyle, d'isononyle, d'isopentyle, d'isobornyle, d'isopulégyle, de linalyle, de menthyle, de méthyl phényl carbinyle, de néryle, de nonyle, d'ortho- (tertio-butyl) cyclohexyle, de phényléthyle, de para-(tertio-butyl) cyclohexyle, de phénéthyle, de prényle, de styrallyle, de terpényle, de 4-tert-butylcyclohexyle ou de vetyvéryle ;
- Les benzoates de benzyle, d'isobutyle ou de linalyle ;
- Les butanoates d'éthyle, de benzyle, ou d'isoamyle ;
- Les butyrates de benzyle, d'éthyle, d'isoamyle ou de lynalyle ;
- Les cinnamates de butyle ou d'éthyle ;
- Les formiates de benzyle, de citronellyle, de géranyle, ou de méthyle ;
- Les isobutyrates de phénoxyéthyle ou de (cis) 3-hexényle ;
- Les propionates d'amyle, d'alkylcyclohexyle, d'allylcyclohexyle, de lynalyle, de styralyle, de citronellyle ;
- Les salicylates de méthyle, de benzyle, ou d'éthyle ;
Ou encore :
- L'anthralinate de méthyle, la coumarine, le tiglate d'hexyle, le caproate d'allyle, l'hédione (dihydrojasmonate de méthyle), le glycinate d'éthylméthylphényle, le glycolate d'allyl-amyle, l'heptanoate d'allyle, le méthacrylate d'isoamyle, le naphtolate d'éthyle ou le néopentanoate d'hexyle.

Parmi les substances parfumantes hydrophobes d'origine synthétique il y a des alcools comme l'alcool benzylique, le 3,7-diméthyl-1-octanol, l'alcool isononylique, l'α-terpinéol, le menthol, le linalol, le citronellol, l'eucalyuptol ou 1,8-cinéole, le géraniol, le phytol, l'iso-phytol, l'α-terpinéol, le tétrahydrolinalol, le farnésol, le carotol, le nérol, le globulol, le vétivérol, le nérolidol, le dihydromyrcénol, le tétrahydromyrcénol, l'alcool fenchylique, le diméthyle benzyle carbinol, l'alcool cinnamique, le 2-phényl éthanol ou l'undécavertol.

Parmi les substances parfumantes hydrophobes d'origine synthétique il y a des dérivés phénoliques comme l'anéthol, le safrol, l'isosafrol, l'eugénol, l'iso-eugénol, le gaïacol ou 2-méthoxy phénol, le chavicol (ou 4-allyl phénol), l'estragol (ou 1-allyl 4-méthoxy benzène), l'alcool cumique, le thymol ou le para-crésol.

Parmi les substances parfumantes hydrophobes d'origine synthétique, il y a des aldéhydes comme le phényl acétaldéhyde, le salicylaldéhyde , l'anisaldéhyde, le capryl aldéhyde, le cinnamaldhéhyde, l'hexyl cinnamaldhéhyde, le bourgeonal, le citral (ou néral), le citronellal, l'hydroxy-citronellal, le citronellyloxyacétaldéhyde, le cyclamènaldéhyde, le cuminaldéhyde, le cyclal, le 2,4-diméthyl-3-cyclohexène-1-carboxaldéhyde (ou ligustral), le dodécanal,, l'éthanal, l'octanal, le décanal, les géranials, l'hélional, le lauraldéhyde, le lilial, le méthyl n-nonyl acétaldéhyde, le méthyl octyl acétaldéhyde, l'undécanal ou la vanilline.

Parmi les substances parfumantes hydrophobes d'origine synthétique, il y a des cétones comme la benzyl acétone, la calone (7-méthyl-2H-benzo-1,5-dioxepin-3(4H)-one), la carvone, le camphre, la civéttone, les damascones, les damascénones, l'éthyl-amyl-cétone, l'éthyl-hexyl-cétone, la géranyl cétone, la jasmone, les irones, le maltol (3-hydroxy-2-methyl-4H-pyran-4-one), l'éthyl maltol, la menthone, l'iso-menthone, la muscone, la méthyl hepténone, les ionones comme la methyl-ionone, la 4-méthylacétophénone, la méthylpentylcétone, la méthyl-heptylcétone, la méthyl-hexylcétone, l'α-isométhylionone ou la méthylcédrylcétone.

Parmi les substances parfumantes hydrophobes d'origine synthétique, il y a des éthers comme l'anéthol, le benzyléthléther, le cédryl methyl ether ou le p-crésyl méthyl éther.

Parmi les substances parfumantes hydrophobes d'origine synthétique, il y a des nitriles comme les triméthyl- 3, 5, 7-octane(ène) nitriles et leurs dérivés α-substitués, le citronellyl nitrile, le citronitrile, le géranyl-nitrile.

Selon un aspect plus particulier, l'invention a pour objet l'utilisation telle que décrite précédemment pour laquelle, la substance parfumante hydrophobe (A) est sélectionnée dans le groupe constitué des huiles essentielles de thym, de sarriette, d'origan, de cannelle de Ceylan, de basilic, de clou de girofle, de Géranium de type Bourbon, de Géranium Egypte, de lavande Aspic, Rosalina, de coriandre, de bois de rose, de fleurs de rose, Palmarosa, de menthe poivrée, de monarde fistuleuse, de Lavandin Super ou de Lavandin Grosso.

L'expression "cosmétiquement acceptable" utilisée dans la définition de la phase aqueuse (P₂) de la composition (C₁) se présentant sous la forme d'une émulsion de type huile-dans-eau, signifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Un milieu cosmétiquement acceptable de la composition (C₁) objet de l'invention peut contenir classiquement de l'eau, un ou plusieurs solvants organiques cosmétiquement acceptables, un mélange d'eau et d'un ou plusieurs solvants organiques. Les solvants cosmétiquement acceptables peuvent plus particulièrement être choisis parmi les alcools polyhydriques comme par exemple le glycérol, le diglycérol, les oligomères du glycérol, l'éthylène glycol, le propylène glycol, l'hexylène glycol, le diéthylène glycol, le xylitol, l'érythritol, le sorbitol, ou les alcools hydrosolubles.

De préférence, la présente invention a pour objet une composition (C₁) telle que définie précédemment et pour laquelle :
- le tensioactif émulsionnant de type eau-dans-huile (S₁) comprend :
   ∘ une composition d'alkylpolyglycosides (C'₁) représentée par la formule (I), dans laquelle le radical R₂ représente le radical 2-octyl dodécanyl-1 obtenu à partir de la suppression du groupe hydroxyl du 2-octyl dodécanol-1 dans la formule (II), et
   ∘ du 2-octyl dodécanol-1 représenté par la formule (III).
- Le tensioactif émulsionnant de type huile-dans-eau (S₂) comprend :
   ∘ au moins un composé de formule (IV), dans laquelle p est égal à 6 et le groupe R₁-(C=O)- représente le radical n-dodécanoyle, et
   ∘ au moins une composition (C'₂) représentée par la formule (VI) dans laquelle le radical R₃ représente au moins un radical choisi parmi les éléments du groupe constitué par le radical n-dodécyle, le radical n-tétradécyle, le radical n-hexadécyle, et
   ∘ optionnellement au moins un alcool gras de formule (VII) dans laquelle R'₃ représente un radical choisi parmi les éléments du groupe constitué par le radical n-dodécyle, n-tétradécyle, n-hexadécyle.
- la substance parfumante hydrophobe (A) est choisie parmi les éléments du groupe constitué par l'Hespéridé Fleuri Chypré, le Fleuri Boisé Fruité, le Fleuri Fruité, la Fougère Fruitée, le Chypre Fruité, le Chypre Fleuri, le Boisé Fruité, l'Ambré Fleuri épicé, l'Ambré Fleuri Fruité.

La présente invention a pour objet une composition (C₁) telle que définie précédemment et pour laquelle le ratio massique du tensioactif émulsionnant de type huile-dans-eau (S₂) sur le tensioactif émulsionnant de type eau-dans-huile (S₁) est compris entre 2,0/1 et 2,8/1, plus particulièrement entre 2,0/1 et 2,6/1.

La composition (C₁) objet de la présente invention telle que définie précédemment est destinée à un usage visant à parfumer la peau, les cheveux, le cuir chevelu, les lèvres, les vêtements ou les linges de maison.

Lorsque la composition (C₁) objet de la présente invention telle que définie précédemment est destinée à un usage visant à parfumer la peau, les cheveux, le cuir chevelu, les lèvres, elle peut en outre comporter des excipients et/ou des principes actifs habituellement mis en oeuvre dans le domaine des formulations à usage topique, en particulier cosmétiques, dermocosmétiques, pharmaceutiques ou dermo-pharmaceutique.

La composition (C₁) objet de la présente invention peut en outre comprendre un ou plusieurs composés auxiliaires choisi parmi les tensioactifs moussants et/ou détergents, les tensioactifs épaississants et/ou gélifiants, les agents stabilisants, les composés filmogènes, les solvants et co-solvants, les agents hydrotropes, les agents plastifiants, les agents émulsionnants et co-émulsionnants, les agents opacificants, les agents nacrants, les agents surgraissants, les séquestrants, les agents chélatants, les agents antioxydants, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents, éventuellement présents dans la composition (C₁) objet de la présente invention, on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer à la composition (C₁) objet de la présente invention, on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras.

Parmi les tensioactifs amphotères moussants et/ou détergents éventuellement présents dans la composition (C₁) objet de la présente invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents éventuellement présents dans la composition (C₁) objet de la présente invention, on peut citer particulièrement les dérivés d'ammoniums quaternaires.

Parmi les tensioactifs non ioniques moussants et/ou détergents éventuellement présents dans la composition (C₁) objet de la présente invention, on peut citer plus particulièrement les alkylpolyglycosides comportant un radical aliphatique, linéaire ou ramifié, saturé ou insaturé, et comportant de 8 à 12 atomes de carbone ; les dérivés d'huile de ricin, les polysorbates, les amides de coprah, les N-alkylamines.

Comme exemples de tensioactifs épaississants et/ou gélifiants éventuellement présents dans la composition (C₁) objet de la présente invention, on peut citer :
- les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120 ;
- les esters gras alcoxylés tels que le PEG 150 pentaérythrytyl tétrastéarate commercialisé sous l'appellation CROTHIX^{™} DS53, le PEG 55 propylene glycol oléate commercialisé sous l'appellation ANTIL^{™} 141 ;
- les carbamates de polyalkylène glycols à chaînes grasses tels que le PPG 14 laureth isophoryl dicarbamate commercialisé sous l'appellation ELFACOS^{™} T211, le PPG 14 palmeth 60 hexyl dicarbamate commercialisé sous l'appellation ELFACOS^{™} GT2125.

Comme exemples d'agents déodorants éventuellement présents dans la composition (C₁) objet de la présente invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2-décanediol ; le 1,3-propanediol ; l'acide salicylique ; le bicarbonate de sodium ; les cyclodextrines ; les zéolithes métalliques ; le Triclosan^{™} ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'agents anti-oxydants éventuellement présents dans la composition (C₁) objet de la présente invention, on peut citer l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S (nom INCI: Tetrasodium Glutamate Diacetate).

Parmi les principes actifs que peut comprendre la composition (C₁) objet de la présente invention, on peut citer:
- les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés); les composés montrant une action apaisante notamment le SEPICALM^{™} S, l'allantoïne et le bisabolol ; les agents anti-inflammatoires ; les composés montrant une action hydratante comme l'urée, les hydroxyurées, le glycérolglucoside, le diglycérolglucoside, les polyglycérylglucosides, le glycérol, le diglycérol, le xylitylpolyglucoside commercialisé sous le nom de marque Aquaxyl^{™}; les extraits végétaux riches en polyphénols comme les extraits de raisin, les extraits de pin, les extraits de vin, les extraits d'olives; les composés montrant une action amincissante ou lipolytique comme la caféine ou ses dérivés, l'ADIPOSLIM^{™}, l'ADIPOLESS^{™}, la fucoxanthine; les protéines N-acylées; les peptides N-acylés comme le MATRIXIL^{™}; les acides aminés N-acylés; les hydrolysâts partiels de protéines N-acylés; les acides aminés; les peptides; les hydrolysâts totaux de protéines; les extraits de soja, par exemple la Raffermine^{™}; les extraits de blé par exemple la TENSINE^{™} ou la GLIADINE^{™}; les extraits végétaux, tels que les extraits végétaux riches en tanins, les extraits végétaux riches en isoflavones ou les extraits végétaux riches en terpènes; les extraits d'algues d'eau douce ou marines; les extraits de plantes marines; les extraits marins en général comme les coraux; les cires essentielles; les extraits bactériens; les céramides; les phospholipides; les composés montrant une action antimicrobienne ou une action purifiante, comme le LIPACIDE^{™} C8G, le LIPACIDE^{™} UG, le SEPICONTROL^{™} A5; l'OCTOPIROX^{™} ou le SENSIVA^{™} SC50; les composés montrant une propriété énergisante ou stimulante comme le PHYSIOGENYL^{™}, le panthénol et ses dérivés comme le SEPICAP^{™} MP; les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™}; le SURVICODE^{™}; les actifs anti-photo vieillissement; les actifs protecteurs de l'intégrité de la jonction dermo-épidermique; les actifs augmentant la synthèse des composants de la matrice extracellulaire comme le collagène, les élastines, les glycosaminoglycanes; les actifs agissant favorablement sur la communication cellulaire chimique comme les cytokines ou physiques comme les intégrines; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés); les actifs améliorant la microcirculation cutanée, par exemple les veinotoniques; les actifs drainants; les actifs à visée décongestionnante comme les extraits de ginko biloba, de lierre, de marron d'inde, de bambou, de ruscus, de petit houx, de centalla asiatica, de fucus, de romarin, de saule; les agents de bronzage ou de brunissement de la peau, comme par exemple la dihydroxyacétone (DHA), l'érythrulose, l'aldéhyde mésotartrique, le glutaraldéhyde, le glycéraldéhyde, l'alloxane, la ninhydrine, les extraits végétaux comme par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683;; les agents connus pour leur action de facilitation et/ou d'accélération du bronzage et/ou du brunissement de la peau humaine, et/ou pour leur action de coloration de la peau humaine, comme par exemple les caraténoïdes ( et plus particulièrement le beta carotène et le gamma carotène), le produit commercialisé sous le nom de marque « Carrot oil » (Nom INCI: Daucus Carota, helianthus annuus Sunflower oil) par la société Provital, qui contient des caroténoïdes, de la vitamine E et de la vitamine K; la tyrosine et/ou ses dérivés, connus pour leur effet sur l'accélération du bronzage de la peau humaine en association avec une exposition aux rayonnements ultra-violets, comme par exemple le produit commercialisé sous le nom de marque « SunTan Accelerator^{™} » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze^{™} (nom INCI: Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI: butylene glycol and Acetyl Tyrosine and hydrolyzed vegetable protein and Adenosine triphosphate) par la société UNIPEX, le produit commercialisé sous le nom de marque « Try-Excell^{™} » (nom INCI: Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze^{™} » (nom INCI: hydrolyzed wheat protein and acetyl tyrosine and copper gluconate) par la société Alban Muller, le produit commercialisé sous le nom de marque Tyrostan^{™} (nom INCI: potassium caproyl tyrosine) par la société Synerga, le produit commercialisé sous le nom de marque Tyrosinol (nom INCI: Sorbitan Isostearate, glyceryl oleate, caproyl Tyrosine) par la société Synerga, le produit commercialisé sous le nom de marque InstaBronze^{™} (nom INCI: Dihydroxyacetone and acetyl tyrosine and copper gluconate) commercialisé par la société Alban Muller, le produit commercialisé sous le nom de marque Tyrosilane (nom INCI: méthylsilanol and acétyl tyrosine) par la société Exymol; les peptides connus pour leur effet d'activation de la mélanogénèse comme par exemple le produit commercialisé sous le nom de marque Bronzing SF Peptide powder (nom INCI: Dextran and Octapeptide-5) par la société Infinitec Activos, le produit commercialisé sous le nom de marque Melitane (nom INCI: Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions^{™} (nom !NC!: Butylene glycol, Palmitoyl Tripeptide-40) par la société LIPOTEC, les sucres et les dérivés de sucres comme par exemple le produit commercialisé sous le nom de marque Tanositol^{™} (nom INCI: inositol) par la société Provital, le produit commercialisé sous le nom de marque Thalitan^{™} (ou Phycosaccharide^{™} AG) par la société CODIF international (nom INCI: Aqua and hydrolyzed algin (Laminaria Digitata) and magnésium sulfate and manganèse sulfate) contenant un oligosaccharide d'origine marine (acide guluronique et acide mannuronique chélatés avec les ions magnésium et manganèse), le produit commercialisé sous le nom de marque Melactiva^{™} (nom INCI: Maltodextrin, Mucuna Pruriens Seed extract) par la société Alban Muller, les composés riches en flavonoïdes comme par exemple le produit commercialisé sous le nom de marque « Biotanning » (nom INCI: Hydrolyzed citrus Aurantium dulcis fruit extract) par la société Silab et connu pour être riche en flavonoides de citron (de type hespéridines).

Parmi les agents de texture que peut comprendre la composition (C₁) objet de la présente invention, on peut citer la lauroyl lysine commercialisée sous l'appellation AMINOHOPE^{™}LL par la société AJINOMOTO, l'octenyl starch succinate commercialise sous l'appellation DRYFLO^{™} par la société NATIONAL STARCH, le myristyl polyglucoside commercialisé par SEPPIC sous l'appellation MONTANOV^{™} 14, les fibres de cellulose, les fibres de coton, les fibres de chitosane.

La composition (C₁) selon l'invention et telle que définie précédemment, est obtenue par la mise en oeuvre du procédé de préparation comprenant les étapes suivantes :
Une étape a) de préparation de la phase grasse (P₁) en mélangeant au moins une huile et optionnellement au moins une cire, au moins un tensioactif émulsionnant de type huile-dans-eau (S₂), au moins une substance parfumante hydrophobe (A) et au moins un tensioactif émulsionnant de type eau-dans-huile (S₁) dans les proportions souhaitées. Cette étape de mélange est généralement conduite à une température supérieure ou égale à 20°C et inférieure ou égale à 80°C, plus particulièrement supérieure ou égale à 25°C et inférieure ou égale à 80°C, et encore plus particulièrement supérieure ou égale à 30°C et inférieure ou égale à 80°C ; elle est réalisée sous agitation mécanique à une vitesse modérée supérieure ou égale à 50 tours/minute et inférieure ou égale à 100 tours/minute ;
Une étape b) d'ajout du polyélectrolyte anionique réticulé (PA) sur le mélange préparé lors de l'étape a). Cette étape de mélange est généralement conduite à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C ; elle est réalisée sous agitation mécanique à une vitesse modérée supérieure ou égale à 500 tours/minute et inférieure ou égale à 3 000 tours/minute.
Une étape c) de préparation de la phase aqueuse (P₂) de l'ensemble des éléments la constituant dans les proportions souhaitées. Cette étape de mélange est généralement conduite à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C ; elle est réalisée sous agitation mécanique à une vitesse supérieure ou égale à 500 tours/minute et inférieure ou égale à 3 000 tours/minute.
Une étape d) au cours de laquelle la phase grasse (P₁) est ajoutée sur la phase aqueuse (P₂) à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C, sous agitation mécanique à une vitesse supérieure ou égale à 2000 tours/minute et inférieure ou égale à 6000 tours/minute, de façon à obtenir la composition selon l'invention.

La présente invention a également pour objet un procédé pour parfumer la peau, les cheveux, le cuir chevelu, les lèvres, les vêtements ou les linges de maison caractérisé en ce qu'il comprend au moins une étape d'application sur ladite peau, lesdits cheveux, ledit cuir chevelu, lesdites lèvres, ledit vêtement ou ledit linge de maison de la composition (C₁) telle que définie précédemment.

Par « linges de maison », on désigne au sens de la présente invention l'ensemble des pièces de tissu destinées à un usage domestique comme par exemple les linges de table comme les nappes, les serviettes de table, les sets de table ; les linges de cuisine et de ménage comme les chiffons, les torchons, les serpillières ; les linges de lit comme les couvertures, les dessus de lit, les draps, les drap-housses, les housses de couettes, les plaids, les taies d'oreiller, les taies de traversin, les couettes, les alèses, les édredons, les oreillers, les traversins ; les linges de bains et/ou de toilettes comme les gants de toilette, les serviettes de bain, les serviettes de plage ; les mouchoirs ; les rideaux et voilages.

La composition (C₁) selon l'invention peut être conditionnée sous forme de flacons, de flacons spray, de flacons pompes, de roll on, de tubes.

L'invention a donc aussi pour objet un contenant, de préférence un flacon, comprenant un moyen de pulvérisation et un moyen de conditionnement, et contenant une composition (C₁) telle que définie précédemment.

Selon un aspect particulier, le moyen de pulvérisation compris dans le contenant objet de la présente invention est une pompe manuelle.

La composition (C₁) selon l'invention peut être ainsi appliquée sous forme de fines particules liquides au moyen de dispositifs de pressurisation. Les dispositifs conformes à l'invention sont bien connus de l'homme du métier et comprennent les pompes non-aérosols ou "atomiseurs", les contenants aérosols comprenant un propulseur ainsi que les pompes.
Les exemples suivants illustrent l'invention, sans toutefois la limiter.

### Préparation et évaluation d'émulsions huile-dans-eau selon l'invention et d'émulsions huile-dans-eau comparatives.

### 1) Préparation des émulsions selon l'invention et des émulsions comparatives

On prépare six émulsions selon l'invention, notées (F₁) à (F₆), et huit émulsions comparatives, notées (E₁) à (E₈), dont les proportions massiques en leurs constituants sont consignées dans le tableau 1 ci-dessous ; les teneurs massiques des polyélectrolytes étant indiquées en pourcentage de matière sèche polymérique, en mettant en oeuvre le procédé ci-après.

Les constituants de la phase grasse sont introduits successivement dans un bêcher, mélangés et amenés à une température de 20°C après une étape de chauffage à 40°C ; le mélange est réalisé à l'aide d'un agitateur mécanique muni d'un mobile d'agitation de type hélice, à une vitesse de 100 tours/minute.

Les constituants de la phase grasse dispersée sont mélangés à température ambiante dans un bêcher à l'aide d'un agitateur mécanique à une vitesse de 2 000 tours/minute et l'agent épaississant est alors ajouté progressivement.

La phase aqueuse est ajoutée en une seule fois sur la phase grasse, à température ambiante et à une vitesse d'agitation de 4 000 tours/minute avec un agitateur équipé d'un mobile cisaillant. Cette agitation est alors maintenue pendant cinq minutes. L'agent conservateur est ensuite ajouté et le mélange résultant est mélangé à température ambiante et à une vitesse d'agitation de 4 000 tours/minute avec un agitateur équipé d'un mobile cisaillant. L'émulsion obtenue est ensuite vidangée.

**Tableau 1 : compositions (F₁), (F₂), (F₃), (F₄), (F₅) et (F₆) selon l'invention**

| | (F₁) | (F₂) | (F₃) | (F₄) | (F₅) | (F₆) |
|---|---|---|---|---|---|---|
| Phase A | | | | | | |
| Eau | 60% | 60% | 60% | 60% | 60% | 60% |
| Fluidifeel Easy ⁽¹⁾ | 1.2% | 1.2% | 1.2% | 1.2% | 1.2% | 1% |
| Emogreen L15 ⁽²⁾ | 2% | 0% | 2% | 0% | 2% | 2% |
| Triglyceride C₈-C₁₀ ⁽³⁾ | 0% | 2% | 0% | 2% | 0% | 0% |
| Parfum « Raspberry »⁽⁴⁾ | 2.5% | 0% | 2.5% | 0% | 2.5% | 2.5% |
| Parfum « Tonic agrume »⁽⁵⁾ | 0% | 2.5% | 0% | 2.5% | 0% | 0% |
| Sepinov^{™}EMT 10 ⁽⁶⁾ | 0.4% | 0.4% | 0% | 0% | 0% | 0% |
| SEPIMAX^{™}Zen⁽⁸⁾ | 0% | 0% | 0.4% | 0.4% | 0.2% | 0.2% |
| Fluidanov^{™}20X ⁽⁷⁾ | 0.5% | 0.5% | 0.5% | 0.5% | 0.5% | 0.5% |
| Ratio Fluidifeel Easy ⁽¹⁾/ Fluidanov^{™}20X ⁽⁷⁾ | 2.4/1 | 2.4/1 | 2.4/1 | 2.4/1 | 2.4/1 | 2/1 |
| Phase B | | | | | | |
| Eau | Qs 100% | Qs 100% | Qs 100% | Qs 100% | Qs 100% | Qs 100% |
| Phase C | | | | | | |
| Euxyl^{™}PE 9010⁽⁹⁾ | 1% | 1% | 1% | 1% | 1% | 1% |

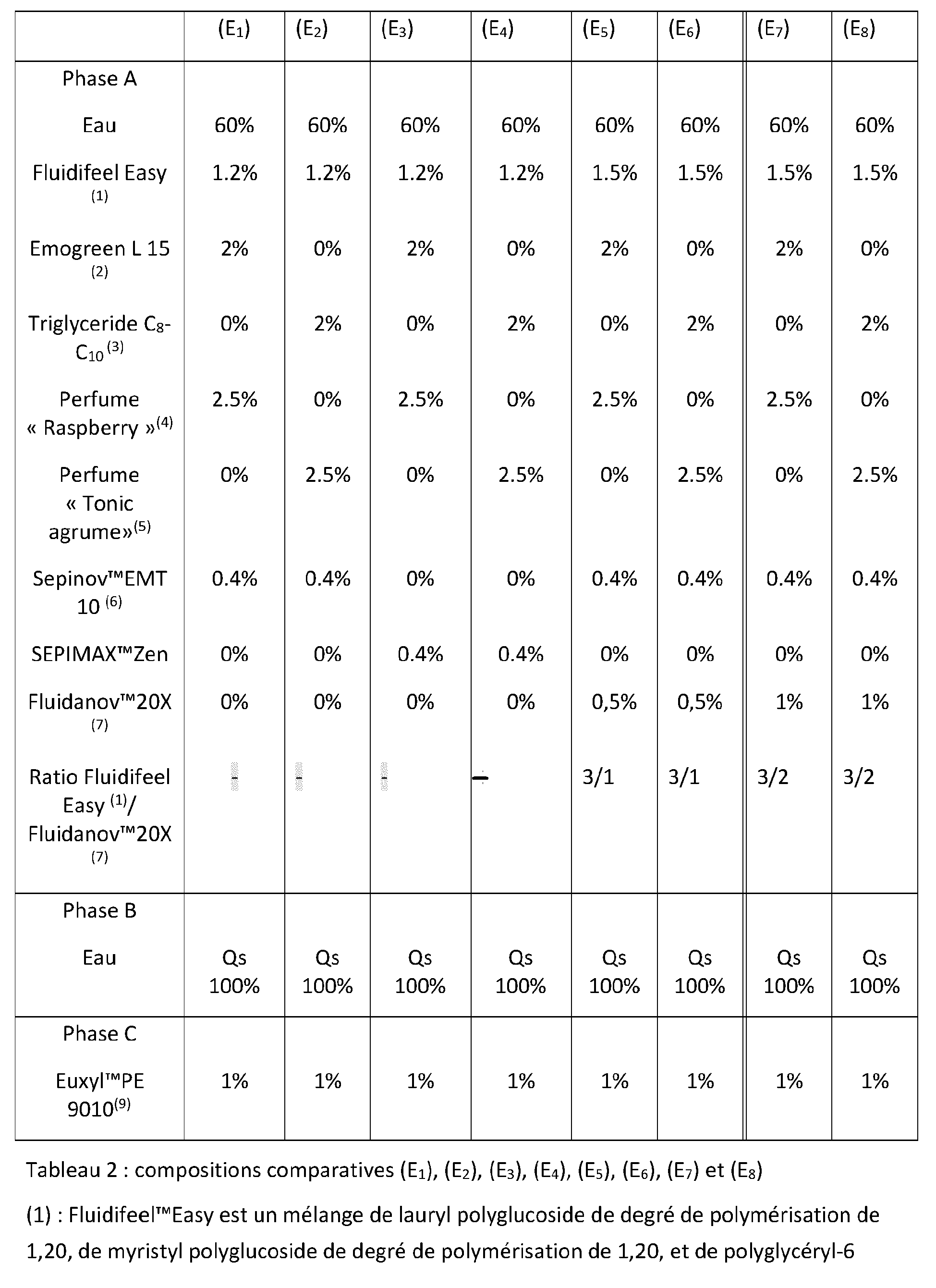

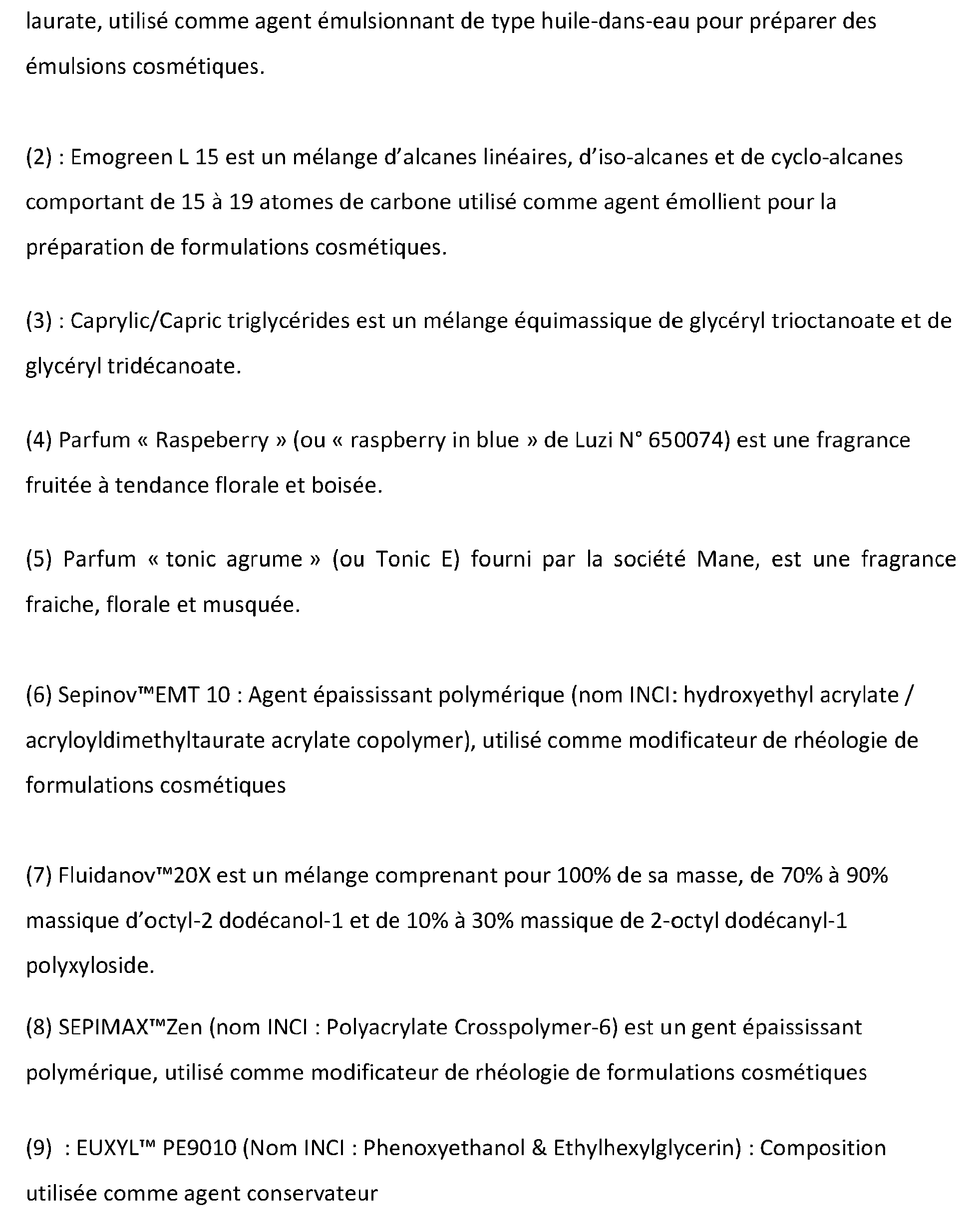

### 2) Evaluation des propriétés des émulsions selon l'invention et des émulsions comparatives

### 2.1 Evaluation de la stabilité des compositions (F₁) à (F₆) selon l'invention et des compositions comparatives (E₁) à (E₈)

### a) On introduit dans une enceinte climatique régulée à 25°C une quantité de 100 mL de la composition à tester et contenue dans un flacon de 250 mL, pendant une durée d'un mois.

On évalue avant la mise en stabilité dans l'enceinte et après une durée d'un mois dans ladite enceinte climatique, l'aspect visuel de la composition testée, qui sera qualifié selon l'observation soit de « Homogène », signifiant une stabilité de la composition testée soit « d'hétérogène », signifiant une instabilité de la composition testée.

### b) On introduit dans une enceinte climatique régulée à 45°C une quantité de 100 mL de la composition à tester et contenue dans un flacon de 250 mL, pendant une durée d'un mois.

On évalue avant la mise en stabilité dans l'enceinte et après une durée d'un mois dans ladite enceinte climatique, l'aspect visuel de la composition testée, qui sera qualifié selon l'observation soit de « Homogène », signifiant une stabilité de la composition testée soit « d'hétérogène », signifiant une instabilité de la composition testée.

### c) Les résultats obtenus sont consignés dans le tableau 3 ci-dessous.

### 2.2 Evaluation du facteur de pulvérisation des compositions (F₁) à (F₆) selon l'invention et des compositions comparatives (E₁) à (E₈)

Principe : l'objectif de ce test est d'apprécier la qualité d'émission de compositions fluides à travers une buse de pulvérisation.

Il s'agit de mesurer la surface d'émission du fluide sur un support cartonné positionné à une distance fixe de l'emballage de pulvérisation, de façon à apprécier qualitativement la pulvérisation du fluide testé.

Ainsi, les fluides qui sont émis sous forme d'un jet, avec une surface de pulvérisation très réduite n'offrent pas une bonne pulvérisation, alors que les fluides émis sous forme d'une surface élevée procurent une qualité élevée de pulvérisation.

### Protocole :

Les produits à testés sont conditionnés dans des flacons d'essais 24 heures avant la réalisation de l'évaluation et stockés dans une enceinte climatique dont la température est régulée à 20°C.

Les flacons en verres de 100 mL sont équipé d'une buse de pulvérisation de type « POMPA AVLP 18/415 » commercialisée par la société Eurovetrocap SPA.

Les chemises cartonnées sont découpées en bandes de 16 centimètres de hauteur et fixées sur un support à une distance de 7 centimètres du corps du flacon pulvérisateur.

Les dispositifs de pulvérisation sont amorcés une fois puis une seconde pression d'amorçage est réalisée avant d'effectuer les mesures.

Les dispositifs de pulvérisation sont placés sur le support à 7 centimètres de la bande de chemise cartonnée, la buse en face de ladite bande de carton.

Deux pressions consécutives sont alors réalisées et observe l'apparition d'une tâche humide sur la bande de carton se présentant sous la forme d'une surface circulaire.

On mesure alors, pour chaque composition, sans prendre en compte les points dispersés :
- la distance L₁ᵢ correspondant à la largeur de la tâche circulaire
- la distance L₂ᵢ correspondant à la largeur de la tâche circulaire

Quatre mesures sont effectuées pour chaque composition testée.

Pour chaque série de compositions dont on souhaite évaluer le facteur de pulvérisation, on réalise également ces mesures avec un fluide témoin adapté, en l'occurrence ici avec de l'eau déminéralisée et à savoir :
- la distance L₁ᵢₑ correspondant à la largeur de la tâche circulaire de l'eau pulvérisée
- la distance L₂ₑ correspondant à la largeur de la tâche circulaire de l'eau pulvérisée.

On calcule ensuite la surface pour chaque composition (i) testée et pour l'eau, puis le « facteur de pulvérisation » comme suit :
- surface composition testée (Si) : Si = L₁ᵢ × L₂ᵢ × π/4
- surface eau (Se) : Se = L₁ₑ × L₂ₑ × π/4
- facteur de pulvérisation (en %) : Fp = (Si/Se) × 100

Dans le cadre de la présente invention concernant des compositions parfumantes, un facteur de pulvérisation (Fp) sera jugé satisfaisant et répondant aux besoins du problème technique s'il est supérieure ou égal à 50%, plus particulièrement supérieur ou égal à 60%.

### 2.3 Résultats.

Les résultats des évaluations obtenus pour les compositions (F₁) à (F₆) selon l'invention sont consignés dans le tableau 3 ci-dessous et ceux obtenus pour les compositions comparatives (E₁) à (E₈) sont consignés dans le tableau 4 ci-dessous.

**Tableau 3 : évaluation de la stabilité et du facteur de pulvérisation des compositions (F₁), (F₂), (F₃), (F₄), (F₅) et (F₆) selon l'invention.**

| | (F₁) | (F₂) | (F₃) | (F₄) | (F₅) | (F₆) |
|---|---|---|---|---|---|---|
| Stabilité un mois 45°C | Liquide homogène | Liquide homogène | Liquide homogène | Liquide homogène | Liquide homogène | Liquide homogène |
| Stabilité un mois 25°C | Liquide homogène | Liquide homogène | Liquide homogène | Liquide homogène | Liquide homogène | Liquide homogène |
| Facteur de pulvérisation (Fp) en % | 80% | 100% | 68% | 79% | 90% | 100% |

**Tableau 4 : évaluation de la stabilité et du facteur de pulvérisation des compositions comparatives (E₁), (E₂), (E₃), (E₄), (E₅), (E₆), (E₇) et (E₈)**

| | (E₁) | (E₂) | (E₃) | (E₄) | (E₅) | (E₆) | (E₇) | (E₈) |
|---|---|---|---|---|---|---|---|---|
| Stabilité un mois 45°C | Hétérogène | Hétérogène | Hétérogène | Hétérogène | Hétérogène | Hétérogène | Hétérogène | Hétérogène |
| Stabilité un mois 25°C | Hétérogène | Stable | Homogène | Homogène | Hétérogène | Hétérogène | Hétérogène | Hétérogène |
| Facteur de pulvérisatio n (Fp) en % | n.m | n.m | n.m | n.m | n.m | n.m | n.m | n.m |

### 2.4 Commentaires.

Les résultats obtenus montrent la nécessité d'inclure un tensioactif émulsionnant de type eau-dans-huile (S₁), en l'occurrence une composition d'octyl-2dodécyl polyxyloside et d'octyl-2dodécanol-1 dans la composition parfumante pour atteindre la stabilité de la composition parfumante à 25°C et 45°C comme montré en comparant (F₁) et (E₁), (F₂) et (E₂), (F₃) et (E₃), (F₄) et (E₄). Ces résultats montrent la présence de cet effet technique stabilisant pour différentes substances parfumées et pour des polélectrolytes anioniques différents (le Sepimax^{™}Zen comprenant un polyélectrolyte anionique réticulé dont le squelette polymérique comprend notamment une unité monomérique de formule (M1) et de nature hydrophobe, alors que le polyélectrolyte anionique réticulé contenu dans le Sepinov^{™}EMT10 est un copolymère réticulé ne comprenant que des unités hydrophiles).

Les compositions selon l'invention se caractérisent également par des facteurs de pulvérisation élevé, certaines d'entre elles étant aussi performante qu'une pulvérisation de l'eau déminéralisée utilisée comme témoins positif.

## Revendications

1. Composition parfumante (C₁) se présentant sous la forme d'une émulsion de type huile-dans-eau et comprenant:
a) Au moins une huile,
b) Au moins un polyélectrolyte anionique réticulé (PA),
c) Au moins un tensioactif émulsionnant de type eau-dans huile (S1) comprenant un alkylpolyxyloside possédant un radical alkyl branché, non linéaire et comportant de 10 à 36 atomes de carbone,
d) Au moins un tensioactif émulsionnant de type huile-dans-eau (S2),
e) Au moins une substance parfumante hydrophobe, et
f) De l'eau ;
Avec le ratio massique du tensioactif émulsionnant de type huile-dans-eau (S₂) sur le tensioactif émulsionnant de type eau-dans-huile (S₁) compris entre 2,0/1 et 2,8/1.

2. Composition selon la revendication 1, **caractérisée en ce qu'**elle comprend pour 100% de sa masse :
a) De 1% à 30% massique d'huile ;
b) De 0,05% à 2,5% massique du polyélectrolyte anionique réticulé ;
c) De 0,05% à 2,5% massique du tensioactif de type eau-dans-huile (S1) ;
d) De 0,5% à 5% massique du tensioactif de type huile-dans-eau (S2) ;
e) De 0,5% à 10% massique de la substance parfumante hydrophobe ;
f) De 50% à 97,9% massique d'eau.

3. Composition selon l'une des revendications 1 ou 2, **caractérisée en ce que** le tensioactif émulsionnant de type eau-dans-huile (S1) comprend au moins une composition d'alkylpolyglycosides (C'1) représentées par la formule (I) :
R₂-O-(X)ₓ-H (I)
dans laquelle x représente un nombre décimal compris entre 1,05 et 2,5, X représente le radical xylosyl ou α,β-D-xylopyranosyl, obtenu à partir de la suppression du groupe hydroxyl hémiacétal du α,β-D-xylopyranose, et R2 représente un radical de formule (II) :
CH(CₜH₂ₜ₊₁)(CᵥH₂ᵥ₊₁)CH₂₋ (II)
dans laquelle t est un nombre entier compris entre 6 et 18, v est un nombre entier compris entre 4 et 18 et la somme t + v est supérieure ou égale à 10 et inférieure ou égale à 22.

4. Composition selon la revendication 3, **caractérisée en ce que** la composition (C'1) consiste en un mélange de composés représentés par les formules (I1), (I2), (I3), (I4) et (I5) :
R2-O-(G)1-H (I1)
R2-O-(G)2-H (I2)
R2-O-(G)3-H (I3)
R2-O-(G)4-H (I4)
R2-O-(G)5-H (I5)
dans les proportions molaires respectives a1, a2, a3, a4 et a5, telles que :
- La somme a1+ a2 + a3 + a4 + a5 est égale à 1 et que
- La somme a1 + 2a2 + 3a3 + 4a4 + 5a5 est égale à x.

5. Composition selon l'une des revendications 3 ou 4, **caractérisée en ce que** le tensioactif émulsionnant de type eau-dans-huile (S1) comprend un alcool gras de formule (III) :
CH(CₜH₂ₜ₊₁)(CᵥH₂ᵥ₊₁)CH₂-OH (III),
dans laquelle t est un nombre entier compris entre 6 et 18, v est un nombre entier compris entre 4 et 18 et la somme t + v est supérieure ou égale à 14.

6. Composition selon l'une des revendications 1 à 5, **caractérisée en ce que** le tensioactif émulsionnant de type huile-dans-eau (S2) comprend :
- Un composé de formule (IV)
R₁-(C=O)-[O-CH₂-CH(OH)-CH₂]ₚ-OH (IV),
dans laquelle p représente un nombre entier supérieur ou égal à 1 et inférieur ou égal à 15, et dans laquelle le groupe R₁-(C=O)- représente un radical acyle, saturé ou insaturé, linéaire ou branché, comportant de six à vingt-deux atomes de carbone, et/ou
- Une composition (C'₂) d'alkylpolyglycosides représentée par la formule (VI) :
R₃-O-(G)x'-H (VI)
dans laquelle x' représente un nombre décimal compris entre 1,05 et 2,5, G représente le radical glucosyl ou α,β-D-glucopyranosyl, obtenu à partir de la suppression du groupe hydroxyl hémiacétal du α,β-D-glucopyranose, et R₃ représente un radical choisi parmi les éléments du groupe constitué par le radical n-dodécyle, n-tétradécyle, n-hexadécyle, n-octadécyle, n-eicosyle et n-béhényle.

7. Composition selon la revendication 6, **caractérisée en ce que** la composition (C'₂) consiste en un mélange de composés représentés par les formules (VI₁), (VI₂), (VI₃), (VI₄) et (VI₅) :
R₃-O-(G)1-H (VI1)
R₃-O-(G)2-H (VI2)
R₃-O-(G)3-H (VI3)
R₃-O-(G)4-H (VI₄)
R₃-O-(G)5-H (VI5)
dans les proportions molaires respectives a1, a2, a3, a4 et a5, telles que :
- La somme a1+ a2 + a3 + a4 + a5 est égale à 1 et que
- La somme a1 + 2a2 + 3a3 + 4a4 + 5a5 est égale à x.

8. Composition selon l'une des revendications 6 ou 7, **caractérisée en ce que** le tensioactif émulsionnant de type huile-dans-eau (S2) comprend un composé de formule (IV) et au moins un composé de formule (V) :
HO-[CH₂-CH(OH)-CH₂-O]ₙ-H (V),
Dans laquelle n, identique ou différent de p, représente un nombre entier supérieur ou égal à 1 et inférieur ou égal à 15.

9. Composition selon l'une des revendications 6 ou 7, **caractérisée en ce que** le tensioactif émulsionnant de type huile-dans-eau (S2) comprend une composition (C'₂) d'alkylpolyglycosides et un alcool gras de formule (VII) :
R'₃-OH (VII),
dans laquelle R'₃ représente un radical choisi parmi les éléments du groupe constitué par le radical n-dodécyle, n-tétradécyle, n-hexadécyle, n-octadécyle, n-eicosyle et n-béhényle, et avec R'₃ identique ou différent de R₃.

10. Composition selon l'une des revendications 3 à 9, **caractérisé en ce que** le radical R2 représente le radical 2-octyl dodécanyl-1 dans la formule (II) obtenu à partir de la suppression du groupe hydroxyl du 2-octyl dodécanol-1, et pour lequel t est égal à 10 et v est égal à 8 dans la formule (II).

11. Composition selon l'une des revendications 1 à 10, **caractérisée en ce que** le polyélectrolyte anionique réticulé (PA) comprend une proportion supérieure ou égale à 25% molaire d'unités monomériques issues de l'acide 2-methy 2-[(1-oxo 2-propènyl) amino] 1-propanesulfonique sous forme acide libre ou partiellement ou totalement salifiée.

12. Composition selon l'une des revendications 1 à 11, **caractérisée en ce que** la substance parfumante hydrophobe (A) est choisie parmi les éléments du groupe constitué par les familles olfactives suivantes : Hespéridé, Florale, Fougère, Chypre, Boisée.

13. Procédé pour parfumer la peau, les cheveux, le cuir chevelu, les lèvres, les vêtements ou les linges de maison **caractérisé en ce qu'**il comprend au moins une étape d'application sur ladite peau, lesdits cheveux, ledit cuir chevelu, lesdites lèvres, ledit vêtement ou ledit linge de maison de la composition (C₁) telle que définie à l'une ou quelconque des revendications 1 à 12.

14. Contenant comprenant un moyen de pulvérisation et un moyen de conditionnement, et contenant une composition (C₁) telle que définie à l'une ou quelconque des revendications 1 à 12.

## Patentansprüche

1. Parfümierungszusammensetzung (C₁), die in Form einer Emulsion vom Öl-in-Wasser-Typ vorliegt und Folgendes umfasst:
a) mindestens ein Öl,
b) mindestens einen vernetzten anionischen Polyelektrolyt (PA),
c) mindestens ein emulgierendes Tensid vom Wasser-in-Öl-Typ (S1), umfassend ein Alkylpolyxylosid mit einem nichtlinearen verzweigten Alkylrest mit 10 bis 36 Kohlenstoffatomen,
d) mindestens ein emulgierendes Tensid vom Öl-in-Wasser-Typ (S2),
e) mindestens eine hydrophobe Parfümierungssubstanz und
f) Wasser;
wobei das Massenverhältnis von emulgierendem Tensid vom Öl-in-Wasser-Typ (S₂) zu emulgierendem Tensid vom Wasser-in-Öl-Typ (S₁) zwischen 2,0/1 und 2,8/1 liegt.

2. Zusammensetzung nach Anspruch 1, **dadurch gekennzeichnet, dass** sie auf 100 % ihrer Masse Folgendes umfasst:
a) 1 bis 30 Massen-% Öl;
b) 0,05 bis 2,5 Massen-% vernetzten anionischen Polyelektrolyt;
c) 0,05 bis 2,5 Massen-% Tensid vom Wasser-in-Öl-Typ (S1);
d) 0,5 bis 5 Massen-% Tensid vom Öl-in-Wasser-Typ (S2);
e) 0,5 bis 10 Massen-% hydrophobe Parfümierungssubstanz;
f) 50 bis 97,9 Massen-% Wasser.

3. Zusammensetzung nach Anspruch 1 oder 2, **dadurch gekennzeichnet, dass** das emulgierende Tensid vom Wasser-in-Öl-Typ (S1) mindestens eine Zusammensetzung von durch die Formel (I) wiedergegebenen Alkylpolyglycosiden (C'1) umfasst:
R₂-O-(X)ₓ-H (I),
wobei x für eine Dezimalzahl zwischen 1,05 und 2,5 steht, X für den durch Weglassung der Hemiacetal-Hydroxylgruppe von α,β-D-Xylopyranose erhaltenen Xylosyl- oder α,β-D-Xylopyranosylrest steht und R₂ für einen Rest der Formel (II) steht:
CH (CₜH₂ₜ₊₁)(CᵥH₂ᵥ₊₁)CH₂- (II),
wobei t eine ganze Zahl zwischen 6 und 18 ist, v eine ganze Zahl zwischen 4 und 18 ist und die Summe t + v größer oder gleich 10 und kleiner oder gleich 22 ist.

4. Zusammensetzung nach Anspruch 3, **dadurch gekennzeichnet, dass** die Zusammensetzung (C'1) aus einem Gemisch von durch die Formeln (I1), (I2), (I3), (I4) und (I5) wiedergegebenen Verbindungen:
R2-O-(G)1-H (I1)
R2-O-(G)2-H (12)
R2-O-(G)3-H (I3)
R2-O-(G)4-H (14)
R2-O-(G)5-H (I5)
in solchen jeweiligen molaren Anteilen a1, a2, a3, a4 und a5, dass:
- die Summe a1 + a2 + a3 + a4 + a5 gleich 1 ist und dass
- die Summe a1 + 2a2 + 3a3 + 4a4 + 5a5 gleich x ist, besteht.

5. Zusammensetzung nach Anspruch 3 oder 4, **dadurch gekennzeichnet, dass** das emulgierende Tensid vom Wasser-in-Öl-Typ (S1) einen Fettalkohol der Formel (III) umfasst:
CH (CₜH₂ₜ₊₁)(CᵥH₂ᵥ₊₁)CH₂-OH (III),
wobei t eine ganze Zahl zwischen 6 und 18 ist, v eine ganze Zahl zwischen 4 und 18 ist und die Summe t + v größer oder gleich 14 ist.

6. Zusammensetzung nach einem der Ansprüche 1 bis 5, **dadurch gekennzeichnet, dass** das emulgierende Tensid vom Öl-in-Wasser-Typ (S2) Folgendes umfasst:
- eine Verbindung der Formel (IV)
R₁-(C=O)-[O-CH₂-CH(OH)-CH₂]ₚ-OH (IV),
wobei p für eine ganze Zahl größer oder gleich 1 und kleiner oder gleich 15 steht und wobei die Gruppe R₁-(C=O)- für einen gesättigten oder ungesättigten, linearen oder verzweigten Acylrest mit sechs bis zweiundzwanzig Kohlenstoffatomen steht, und/oder
- eine Zusammensetzung (C'₂) von durch die Formel (VI) wiedergegebenen Alkylpolyglycosiden:
R₃-O-(G)x'-H (VI),
wobei x' für eine Dezimalzahl zwischen 1,05 und 2,5 steht, G für den durch Weglassung der Hemiacetal-Hydroxylgruppe von α,β-D-Glucopyranose erhaltenen Glucosyl- oder α,β-D-Glucopyranosylrest steht und R₃ für einen Rest steht, der aus den Elementen der Gruppe bestehend aus n-Dodecyl-, n-Tetradecyl-, n-Hexadecyl, n-Octadecyl-, n-Eicosyl- und n-Behenylresten ausgewählt ist.

7. Zusammensetzung nach Anspruch 6, **dadurch gekennzeichnet, dass** die Zusammensetzung (C'₂) aus einem Gemisch von durch die Formeln (VI₁), (VI₂), (VI₃), (VI₄) und (VI₅) wiedergegebenen Verbindungen:
R₃-O-(G)1-H (VI1)
R₃-O-(G)2-H (VI2)
R₃-O-(G) 3-H (VI3)
R₃-O-(G) 4-H (VI4)
R₃-O-(G) 5-H (VI5)
in solchen jeweiligen molaren Anteilen a1, a2, a3, a4 und a5, dass:
- die Summe a1 + a2 + a3 + a4 + a5 gleich 1 ist und dass
- die Summe a1 + 2a2 + 3a3 + 4a4 + 5a5 gleich x ist, besteht.

8. Zusammensetzung nach Anspruch 6 oder 7, **dadurch gekennzeichnet, dass** das emulgierende Tensid vom Ölin-Wasser-Typ (S2) eine Verbindung der Formel (IV) und mindestens eine Verbindung der Formel (V):
HO-[CH₂-CH(OH)-CH₂-O]n-H (V)
umfasst, wobei n mit p identisch oder davon verschieden ist und für eine ganze Zahl größer oder gleich 1 und kleiner oder gleich 15 steht.

9. Zusammensetzung nach Anspruch 6 oder 7, **dadurch gekennzeichnet, dass** das emulgierende Tensid vom Ölin-Wasser-Typ (S2) eine Zusammensetzung (C'₂) von Alkylpolyglycosiden und einen Fettalkohol der Formel (VII):
R'₃-OH (VII)
umfasst, wobei R'₃ für einen Rest steht, der aus den Elementen der Gruppe bestehend aus n-Dodecyl-, n-Tetradecyl-, n-Hexadecyl, n-Octadecyl-, n-Eicosyl- und n-Behenylresten ausgewählt ist, und wobei R'₃ mit R₃ identisch oder davon verschieden ist.

10. Zusammensetzung nach einem der Ansprüche 3 bis 9, **dadurch gekennzeichnet, dass** der Rest R2 in der Formel (II) für den durch Weglassung der Hydroxylgruppe von 2-Octyl-1-dodecanol erhaltenen 2-Octyl-1-dodecanylrest, für den in der Formel (II) t gleich 10 ist und v gleich 8 ist, steht.

11. Zusammensetzung nach einem der Ansprüche 1 bis 10, **dadurch gekennzeichnet, dass** der vernetzte anionische Polyelektrolyt (PA) einen Anteil größer oder gleich 25 Mol-% von Monomereinheiten, die sich von 2-Methyl-2-[(1-oxo-2-propenyl)amino]-1-propan-sulfonsäure in Form der freien Säure oder teilweise oder vollständig versalzter Form ableiten, umfasst.

12. Zusammensetzung nach einem der Ansprüche 1 bis 11, **dadurch gekennzeichnet, dass** die hydrophobe Parfümierungssubstanz (A) aus den Elementen der Gruppe bestehend aus den folgenden Geruchsfamilien ausgewählt ist: Zitrus, Blumig, Fougäre, Chypre, Holzig.

13. Verfahren zum Parfümieren von Haut, Haaren, Kopfhaut, Lippen, Kleidungsstücken oder Hauswäsche, **dadurch gekennzeichnet, dass** es mindestens einen Schritt des Aufbringens der Zusammensetzung (C₁) gemäß einem oder irgendeinem der Ansprüche 1 bis 12 auf die Haut, die Haare, die Kopfhaut, die Lippen, das Kleidungsstück oder die Hauswäsche umfasst.

14. Behälter, der ein Sprühmittel und ein Verpackungsmittel umfasst und eine Zusammensetzung (C₁) gemäß einem oder irgendeinem der Ansprüche 1 bis 12 enthält.

## Claims

1. Perfuming composition (C₁) provided in the form of an emulsion of oil-in-water-type and comprising:
a) at least one oil,
b) at least one crosslinked anionic polyelectrolyte (PA),
c) at least one emulsifying surfactant of water-in-oil type (S1) comprising an alkyl polyxyloside possessing a nonlinear branched alkyl radical and comprising from 10 to 36 carbon atoms,
d) at least one emulsifying surfactant of oil-in-water type (S2),
e) at least one hydrophobic perfuming substance, and
f) water;
with a ratio by weight of the emulsifying surfactant of oil-in-water type (S₂) to the emulsifying surfactant of water-in-oil type (S₁) of between 2.0/1 and 2.8/1.

2. Composition according to Claim 1, **characterized in that** it comprises, per 100% of its weight:
a) from 1% to 30% by weight of oil;
b) from 0.05% to 2.5% by weight of crosslinked anionic polyelectrolyte;
c) from 0.05% to 2.5% by weight of the surfactant of water-in-oil type (S1);
d) from 0.5% to 5% by weight of the surfactant of oil-in-water type (S2);
e) from 0.5% to 10% by weight of the hydrophobic perfuming substance;
f) from 50% to 97.9% by weight of water.

3. Composition according to either of Claims 1 and 2, **characterized in that** the emulsifying surfactant of water-in-oil type (S1) comprises at least one composition of alkyl polyglycosides (C'1) represented by the formula (I) :
R₂-O-(X)ₓ-H (I)
in which x represents a decimal number of between 1.05 and 2.5, X represents the xylosyl or α,β-D-xylopyranosyl radical, obtained from the deletion of the hemiacetal hydroxyl group of α,β-D-xylopyranose, and R₂ represents a radical of formula (II):
CH(CₜH₂ₜ₊₁) (CᵥH₂ᵥ₊₁)CH₂₋ (II)
in which t is an integer of between 6 and 18, v is an integer of between 4 and 18 and the sum t + v is greater than or equal to 10 and less than or equal to 22.

4. Composition according to Claim 3, **characterized in that** the composition (C'1) consists of a mixture of compounds represented by the formulae (I1), (I2), (I3), (I4) and (I5):
R2-O-(G)1-H (I1)
R2-O-(G)2-H (I2)
R2-O-(G)3-H (I3)
R2-O-(G)4-H (I4)
R2-O-(G)5-H (I5)
in the respective molar proportions a1, a2, a3, a4 and a5, such that:
- the sum a1 + a2 + a3 + a4 + a5 is equal to 1, and that
- the sum a1 + 2a2 + 3a3 + 4a4 + 5a5 is equal to x.

5. Composition according to either of Claims 3 and 4, **characterized in that** the emulsifying surfactant of water-in-oil type (S1) comprises a fatty alcohol of formula (III):
CH (CₜH₂ₜ₊₁) (CᵥH₂ᵥ₊₁) CH₂-OH (III),
in which t is an integer of between 6 and 18, v is an integer of between 4 and 18 and the sum t + v is greater than or equal to 14.

6. Composition according to one of Claims 1 to 5, **characterized in that** the emulsifying surfactant of oil-in-water type (S2) comprises:
- a compound of formula (IV)
R₁-(C=O)-[O-CH₂-CH(OH)-CH₂]ₚ-OH (IV),
in which p represents an integer greater than or equal to 1 and less than or equal to 15 and in which the group R₁-(C=O)- represents a saturated or unsaturated, linear or branched acyl radical comprising from six to twenty-two carbon atoms, and/or
- a composition (C'₂) of alkyl polyglycosides represented by the formula (VI):
R₃-O-(G)x'-H (VI)
in which x' represents a decimal number of between 1.05 and 2.5, G represents the glucosyl or α,β-D-glucopyranosyl radical, obtained from the deletion of the hemiacetal hydroxyl group of α,β-D-glucopyranose, and R₃ represents a radical chosen from the elements of the group consisting of n-dodecyl, n-tetradecyl, n-hexadecyl, n-octadecyl, n-eicosyl and n-behenyl radicals.

7. Composition according to Claim 6, **characterized in that** the composition (C'₂) consists of a mixture of compounds represented by formulae (VI₁), (VI₂), (VI₃), (VI₄) and (VI₅):
R₃-O-(G)1-H (VI1)
R₃-O-(G)2-H (VI2)
R₃-O-(G)3-H (VI3)
R₃-O-(G)4-H (VI4)
R₃-O-(G)5-H (VI5)
in the respective molar proportions a1, a2, a3, a4 and a5, such that:
- the sum a1 + a2 + a3 + a4 + a5 is equal to 1, and that
- the sum a1 + 2a2 + 3a3 + 4a4 + 5a5 is equal to x.

8. Composition according to either of Claims 6 and 7, **characterized in that** the emulsifying surfactant of oil-in-water type (S2) comprises a compound of formula (IV) and at least one compound of formula (V):
HO-[CH₂-CH(OH)-CH₂-O]ₙ-H (V),
in which n, which is identical to or different from p, represents an integer of greater than or equal to 1 and less than or equal to 15.

9. Composition according to either of Claims 6 or 7, **characterized in that** the emulsifying surfactant of oil-in-water type (S2) comprises a composition (C'₂) of alkyl polyglycosides and a fatty alcohol of formula (VII):
R'₃-OH (VII),
in which R'₃ represents a radical chosen from the elements of the group consisting of the n-dodecyl, n-tetradecyl, n-hexadecyl, n-octadecyl, n-eicosyl and n-behenyl radicals, and with R'₃ identical to or different from R₃.

10. Composition according to one of Claims 3 to 9, **characterized in that** the R2 radical represents the 2-octyl-1-dodecyl radical in the formula (II) obtained from the deletion of the hydroxyl group of 2-octyl-1-dodecanol, and for which t is equal to 10 and v is equal to 8 in the formula (II).

11. Composition according to one of Claims 1 to 10, **characterized in that** the crosslinked anionic polyelectrolyte (PA) comprises a proportion of greater than or equal to 25 mol% of monomer units resulting from 2-methyl-2-[(1-oxo-2-propenyl)amino]-1-propanesulfonic acid in free acid or partially or completely salified form.

12. Composition according to one of Claims 1 to 11, **characterized in that** the hydrophobic perfuming substance (A) is chosen from the elements of the group consisting of the following olfactory families: Citrus, Floral, Fougère, Chypre, Woody.

13. Process for perfuming the skin, hair, scalp, lips, clothes or household linen, **characterized in that** it comprises at least one stage of application to said skin, hair, scalp, lips, clothes or household linen of the composition (C₁) as defined in one or any of Claims 1 to 12.

14. Container comprising a spraying means and a packaging means, and containing a composition (C₁) as defined in one or any of Claims 1 to 12.
